(19) Europäisches Patentamt
European Patent Office
Office européen des brevets

(11) **EP 2 892 442 B1**

(12) **EUROPÄISCHE PATENTSCHRIFT**

(45) Veröffentlichungstag und Bekanntmachung des Hinweises auf die Patenterteilung:
**11.07.2018 Patentblatt 2018/28**

(21) Anmeldenummer: **13752902.0**

(22) Anmeldetag: **26.08.2013**

(51) Int Cl.:
*A61B 17/16* (2006.01)   *A61B 17/32* (2006.01)
*B23B 31/00* (2006.01)   *B23B 31/107* (2006.01)

(86) Internationale Anmeldenummer:
**PCT/EP2013/067641**

(87) Internationale Veröffentlichungsnummer:
**WO 2014/037239 (13.03.2014 Gazette 2014/11)**

(54) **CHIRURGISCHES, DREHMOMENT ÜBERTRAGENDES INSTRUMENT EINSCHLIESSLICH ZUGEHÖRIGEM WERKZEUG**

SURGICAL, TORQUE-TRANSMITTING INSTRUMENT INCLUDING ASSOCIATED TOOL

INSTRUMENT CHIRURGICAL TRANSMETTANT UN COUPLE ET COMPRENANT UN OUTIL CORRESPONDANT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **05.09.2012 DE 102012108266**

(43) Veröffentlichungstag der Anmeldung:
**15.07.2015 Patentblatt 2015/29**

(73) Patentinhaber: **Aesculap AG**
**78532 Tuttlingen (DE)**

(72) Erfinder:
• **BARTH, Jürgen**
**78588 Denkingen (DE)**
• **KRAFT, Florian**
**78579 Neuhausen ob Eck (DE)**

(74) Vertreter: **Winter, Brandl, Fürniss, Hübner, Röss, Kaiser, Polte - Partnerschaft mbB Patent- und Rechtsanwaltskanzlei Alois-Steinecker-Strasse 22 85354 Freising (DE)**

(56) Entgegenhaltungen:
EP-A1- 0 056 266    CH-A5- 686 113
DE-A1- 19 918 638    DE-A1-102012 101 259
DE-B3- 10 311 455    US-A1- 2003 023 256
US-A1- 2011 098 688

## Beschreibung

Die vorliegende Erfindung betrifft ein chirurgisches Instrument für das Bereitstellen eines Drehmoments sowie ein angetriebenes, drehbar im Instrumentenhandstück bzw. daran angeschlossenen Handstückschaft gelagertes Werkzeug, auf das ein möglichst hohes Drehmoment übertragbar ist.

## Hintergrund der Erfindung

In der modernen (minimal invasiven) Chirurgie werden Instrumente beispielsweise zur spanabhebenden bzw. materialabtragenden Bearbeitung von Knochen, Knorpeln etc., beispielsweise bei arthroskopischen Eingriffen, in der Wirbelsäulenchirurgie und dergleichen orthopädischen Behandlungen verwendet, welche ein ergonomisch geformtes Handstück und ein ggf. austauschbares Werkzeug (z.B. ein Fräser, Drehmesser, Polierkopf, etc.) umfassen, das im Handstück an dessen distalem Ende drehbar sowie angetrieben gelagert ist. Als Werkzeugantrieb ist je nach Verwendungszweck und beabsichtigter Werkzeugdrehzahl ein hydraulischer, pneumatischer oder elektromotorischer Antrieb vorgesehen, der über einen Drehmoment-Übertragungszug (beispielsweise ein Getriebe und/oder eine Anzahl ggf. miteinander gekoppelter Wellen) innerhalb des Handstücks mit dem Werkzeug wirkverbunden ist. Die Antriebe können dabei im Handstück integriert sein oder sie sind als externe Antriebseinheiten ausgebildet, welche über Energieversorgungsleitungen oder Drehmoment-Übertragungsstränge (z.B. biegeelastische Wellen) mit dem Handstück gekoppelt sind, wobei in diesem Fall das Handstück im Wesentlichen lediglich zur Unterbringung des Getriebes oder Drehmoment-Übertragungszugs dient.

An den Handstücken sind an deren körperzugewandten (distalen) Enden in der Regel rohrförmige Handstückschäfte angeschlossen/montiert, die je nach Einsatzzweck unterschiedliche Schaftlängen und-formen haben, um an unterschiedliche Stellen innerhalb eines Patientenkörpers vorzudringen. So existieren beispielsweise gerade, bogenförmig gekrümmte oder vorzugsweise im Montagebereich mit dem Handstück gekröpfte (abgewinkelte) Handstückschäfte, in denen jedoch immer eine Drehmoment-Übertragungswelle oder - schaft (nachfolgend Torsionswelle) gelagert ist. Diese Welle/Schaft muss starr (torsionssteif) genug sein, um das erforderliche Drehmoment an ein daran distal eingesetztes / ausgebildetes Werkzeug übertragen zu können (d.h. die Welle muss eine ausreichende Torsionssteifigkeit besitzen), jedoch biegsam genug sein, d.h. auch eine gewisse Biegeflexibilität aufweisen, um den Krümmungen eines (nicht geraden) Handstückschaft-Verlaufs auch bei einer drehenden Bewegung folgen zu können.

Zur Verbindung des Werkzeugs mit der im Handstückschaft gelagerten Torsionswelle ist eine Wellenkupplung zur lösbaren Aufnahme eines Werkzeugschafts vorgesehen. Eine Schwierigkeit besteht hierbei jedoch darin, eine derartige Wellenkupplung für das ggf. austauschbare Werkzeug innerhalb des kleindurchmessrigen Handstückschafts so zu gestalten, dass auch bei derart kleinen Handstückschaft-Durchmessern und hohen Drehzahlen eine sichere und langlebige Funktion des chirurgischen Instruments insbesondere auch im Fall von langen Handstückschäften gewährleistet ist. Außerdem sollte der Handstückschaft ebenfalls austauschbar am Handstück aufgenommen sein, um unterschiedliche Schaftlängen und - formen mit einem einzigen Handstück realisieren zu können. Entscheidend hierfür ist die weitere lösbare Drehmomentverbindung zwischen dem im Handstück untergebrachten Getriebe / Drehmomentübertragungszug und der im Schaft gelagerten Torsionswelle, welche zum Einen einfach und sicher schließbar sein muss und zum Anderen ausreichend hohe Drehmomente übertragen sollte. Schließlich sollte die Bedienbarkeit des Instruments einschließlich des Wechsels eines Werkzeugs und/oder eines Handstückschafts einfach und sicher sein.

## Stand der Technik

Beispielsweise aus der EP1605837 A1 ist ein chirurgisches Instrument dieser Gattung und insbesondere ein Handstück eines solchen chirurgischen Instruments bekannt. Das bekannte Handstück besteht in diesem besonderen Fall aus einem hülsenförmigen Griffabschnitt (könnte natürlich auch eine andere Griffform haben), an dessen proximalem (körperabgewandtem) Ende ein Leitungspaket zur Energieversorgung (Druckluft, elektrischer Strom oder Hydraulikdruck) anschließbar ist und an dessen distalem (körperzugewandtem) Ende ein Handstückschaft mittels einer Überwurfmutter (ggf. auswechselbar) aufgeschraubt ist. Der Handstückschaft hat eine äußere sowie innere Schafthülle, die auch zur Gleit-Drehführung einer darin eingeführten Torsionswelle dient. Die innere Schafthülle ist in Axialrichtung in mehrere Teilabschnitte unterteilt, zwischen denen jeweils ein Kugellager in die äußere Schafthülle eingesetzt ist, welche die Torsionswelle gegen die äußere Schafthülle abstützen. Am distalen Ende der Torsionswelle ist ein Werkzeug, vorzugsweise ein Fräskopf fixiert/ausgebildet.

Wie aus dieser Druckschrift zu entnehmen ist, wird das Werkzeug grundsätzlich aus einem Eingriffs- bzw. Schneidkopf und der Torsionswelle gebildet, die einstückig miteinander verbunden sind. Die Kopplung zwischen Werkzeug und Getriebe/Drehmomentübertragungszug innerhalb des Griffabschnitts erfolgt demnach ausschließlich im Bereich der Überwurfmutter. Dies bedeutet, dass das Werkzeug eine speziell an diesen einen Handstückschaft längenangepasste Einzelanfertigung ist, die nicht für andere Handstückschäfte unterschiedlicher Längen verwendbar ist. Es ist daher offensichtlich, dass ein solches Konstruktionsprinzip teuer in der Herstellung wie auch in der Bereitstellung ist, da für jeden Handstückschaft passendes Werkzeug vorhanden/vorgehalten sein muss.

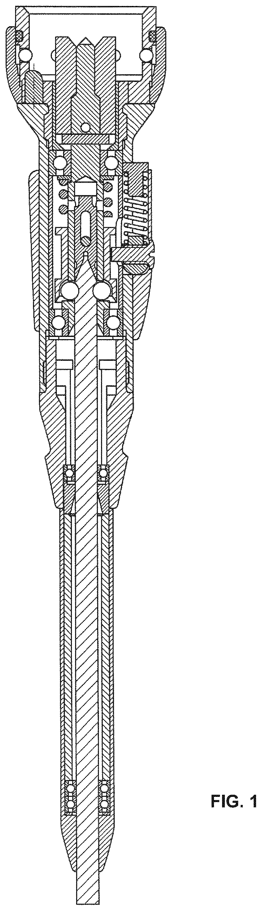

Aus der anliegenden Fig. 1 ist der Längsschnitt eines solchen bekannten chirurgischen Instruments mit bereits eingesetztem Werkzeug schematisch dargestellt.

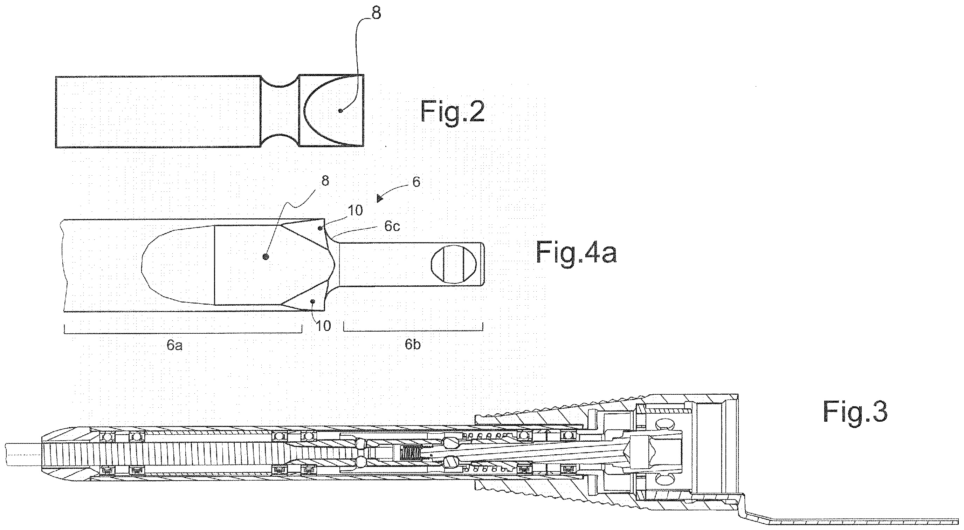

Demnach hat das bekannte Werkzeug den Werkzeugschaft, der aus dem distalen Ende des Instrumenten-/Handstückschafts drehbar herausragt und an dessen distalem Ende ein (nicht weiter dargestellter) Schneidkopf ausgebildet ist. Am in der Fig. 2 in Vergrößerung dargestellten proximalen Werkzeugschaftende ist der bekannte Werkzeugschaft keilförmig angeschärft, wodurch zwei voneinander abgewandte Schrägflächen zur Drehmomenteinleitung ausgebildet werden (entspricht einem sogenannten Zweiflach). Am distalen Endbereich dieser Keilform ist der Werkzeugschaft mit einer umlaufenden Nut ausgeformt, welche als Axialsicherung dient, wie dies nachfolgend noch beschrieben wird.

Das bekannte Handstück ist entsprechend der vorstehenden Werkzeugkonstruktion an seinem distalen Ende mit einer axial verschiebbaren Überwurfhülse versehen, über die der Instrumenten-/Handstückschaft am Handstück drehfest ankoppelbar ist. Innerhalb des Handstücks im Bereich der Überwurfhülse ist ein drehbar gelagertes Aufnahmerohr vorgesehen, das an seinem proximalen Endabschnitt wiederum in einer Drehwelle steckt und dort mittels eines Querstifts drehfest gesichert ist.

Am distalen Ende des Aufnahmerohrs sind zumindest zwei diametral sich gegenüberliegende Bohrungen ausgeformt, in welche Klemmkugeln beweglich eingesetzt sind. Um die Außenseite des Aufnahmerohrs ist eine Schließ- oder Klemmhülse axialverschiebbar gelagert, die in einer ersten Axialposition die Klemmkugeln für eine Bewegung radial nach Außen freigibt und in einer zweiten Axialposition die Klemmkugeln radial nach innen drückt. Zur manuellen Betätigung der Klemmhülse ist ein weiterer Schieber vorgesehen, der an der Außenseite des Handstücks gelagert und über einen Mitnahmestift mit der Klemmhülse verbunden ist. Dabei sei noch erwähnt, dass der Schieber in Richtung zweiter Axialposition der Klemmhülse federvorgespannt ist.

Wie ferner aus der Fig. 1 zu ersehen ist, befindet sich innerhalb des Aufnahmerohrs ein axial relativverschiebbarer Drehmoment-Übertragungsbolzen mit einer distal angeordneten, axial sich erstreckenden, keilförmigen Kerbe, die mit der Keilform des Werkzeugschafts in Drehmoment-Übertragungseingriff bringbar ist. Der Bolzen ist dabei mittels einer Feder in Richtung distal vorgespannt und durch einen Querstift drehfest im Aufnahmerohr gesichert.

Gemäß diesem konstruktiven Aufbau muss das bekannte Werkzeug mit seinem Werkzeugschaft voraus an der distalen Spitze des Handstückschafts in diesen eingeführt und in Richtung zum Aufnahmerohr axial darin verschoben werden, bis der proximale Werkzeugschaftkeil (Zweiflach) an den Klemmkugeln anliegt. Jetzt wird die Klemmhülse über den Schieber in deren Freigabeposition axial verschoben, sodass der Werkzeugschaftkeil die Klemmkugeln radial nach Außen verdrängen und so weiter in das Aufnahmerohr einfahren kann, bis er in der Kerbe des Drehmoment-Übertragungsbolzens zu liegen kommt. Wird jetzt der Schieber wieder freigegeben. Dabei bewegt sich die Klemmhülse eigenständig (angetrieben durch die Vorspannfeder) in deren Klemmposition zurück, in welcher die Klemmkugeln radial nach Innen in die Umfangsnut am Werkzeugschaft gedrückt werden und damit den Werkzeugschaft axial verriegeln. Auf diese Weise kann nunmehr ein Drehmoment von der Drehwelle über den Querstift, das Aufnahmerohr, den weiteren Querstift und den Drehmoment-Übertragungsbolzen (Drehmoment-Übertragungszug) auf den Werkzeugschaft gebracht werden.

Die vorstehend beschriebene, bekannte Konstruktion hat jedoch einige verbesserungswürdige Besonderheiten: Die beschriebenen Querstiftverbindungen bewirken eine lokale Materialschwächung am Drehmoment-Übertragungsbolzen und an dem Aufnahmerohr wie auch an der Drehwelle innerhalb des Handstücks. Außerdem sind die Querstifte relativ dünn und brechen daher schnell. Insgesamt ist daher das übertragbare Drehmoment eingeschränkt.

Die Keilform des Werkzeugschafts ist ebenfalls nicht gut geeignet, hohe Drehmomente zu übertragen, da der axial wirkende Kraftanteil ein Lösen der Werkzeugseitigen Keilform von der Kerbe des Drehmoment-Übertragungsbolzens bewirkt. Darüber hinaus ist mit einem Aufspreizen des Aufnahmerohrs im Bereich der Kerbe zu rechnen.

Schließlich ist die gesamte Kupplungsmechanik zur Verbindung des Werkzeugschafts mit dem Getriebe/Drehmomentübertragungszugs innerhalb des InstrumentenHandstücks in den Bereich der Überwurfhülse verlagert, wo ggf. noch ausreichend radialer Platz zur Aufnahme der Kupplungselemente besteht. Dadurch müssen aber die Werkzeugschäfte die gesamte Länge der Instrumenten-/Handstückschäfte überbrücken. D.h. für jeden Handstückschaft sind spezielle Werkzeuge erforderlich.

Die US 2011/098688 A1 offenbart ein spanabhebendes Werkzeug eines chirurgischen Instruments, nämlich eine Säge ohne Drehmoment-Übertragung. In mehreren Textstellen der US 2011/098688 A1 wird beschrieben, dass das Werkzeug gegen eine radiale Bewegung gesichert bzw. gesperrt ist

Die US 2003/0023256 A1 offenbart den Oberbegriff von Anspruch 1 und wird als nächstliegender Stand der Technik angesehen. Schließlich ist die DE 103 11 455 B3 als technologischer Hintergrund für die vorliegende Erfindung zu erwähnen. Nach dieser Lehre sind die Funktionsabschnitte "Axialverriegelung" und "Drehmoment-Übertragung" nicht erfindungsgemäß, sondern genau umgekehrt dazu angeordnet.

**Kurzbeschreibung der Erfindung**

Angesichts dieses Stands der Technik ist es die Aufgabe der vorliegenden Erfindung, ein Werkzeug für ein chirurgisches, Drehmoment übertragendes Instrument sowie ein System (chirurgisches Instrument) vorzugsweise aus einem Instrumentenhandstück und zumindest einem (oder mehreren) Werkzeug(en) gemäß der Erfindung bereit zu stellen, mit welchem jeweils eine höhere Funktionalität erreichbar ist. Vorzugsweise soll das Instrument insgesamt hohe Drehmomente übertragen können und weiter vorzugsweise einfach und sicher bedienbar sein. Ein Ziel ist es dabei, wenn durch die Verwendbarkeit universeller Werkzeuge für unterschiedliche (auswechselbare) Handstückschäfte die Herstellungs- und Bereithaltungskosten für das System/Instrument gesenkt werden könnten.

Des weiteren ist es beabsichtigte Aufgabe der Erfindung, den verklemmfreien Einsatz des Werkzeugs in die Werkzeugaufnahme zu verbessern.

Die vorstehende Aufgabe sowie die weiteren vorteilhaften Ziele der Erfindung werden durch ein Werkzeug für ein gattungsgemäßes chirurgisches Instrument mit den Merkmalen des Anspruchs 1 sowie ein chirurgisches, Drehmoment übertragendes Instrument mit dem erfindungsgemäßen Werkzeug und den Merkmalen des Anspruchs 6 gelöst. Vorteilhafte Ausgestaltungen und/oder Weiterbildungen der Erfindung sind Gegenstand der Unteransprüche.

Der Kern der vorliegenden Erfindung besteht demzufolge in der Bereitstellung eines Werkzeugs, das die Übertragung eines höheren Drehmoments ohne Vergrößerung des radialen Bauraums zulässt und damit die Grundlage für die Anordnung einer Werkzeugaufnahme innerhalb des (austauschbaren) Handstückschafts des chirurgischen Instruments schafft. Erreicht wird dies zunächst prinzipiell dadurch, dass der für ein Drehmoment übertragendes Einsetzen in die Werkzeugaufnahme des Instruments vorbereitete proximale Endabschnitt des Werkzeugschafts zumindest in einen Funktionsabschnitt "Drehmoment-Übertragung" und einen axial hiervon beabstandeten (unabhängigen) Funktionsabschnitt "Axialverriegelung" unterteilt ist, wobei sich der Funktionsabschnitt "Axialverriegelung" erfindungsgemäß proximal zum Funktionsabschnitt "Drehmoment-Übertragung" anordnet (d.h., der Funktionsabschnitt "Drehmoment-Übertragung befindet sich axial zwischen dem Funktionsabschnitt "Axialverriegelung" und dem Instrumentenkopf). Dadurch wird der Funktionsabschnitt "Axialverriegelung" aus dem Drehmomentfluss genommen und kann daher ausschließlich zum Zweck der Axialverriegelung dimensioniert werden. Außerdem führen alle konstruktiven Maßnahmen zur Erfüllung dieser Funktion zu keiner Beeinflussung/Schwächung der im Drehmomentfluss befindlichen übrigen Schaftabschnitte, sodass das maximal übertragbare Drehmoment gesteigert werden kann bzw. der für den Funktionsabschnitt "Axialverriegelung" radialen Bauraum klein sein kann.

Vorteilhaft ist es, wenn der Werkzeugschaftdurchmesser d im Funktionsabschnitt "Axialverriegelung" kleiner ist als der Werkzeugschaftdurchmesser D im Funktionsabschnitt "Drehmoment-Übertragung". Dies schafft radialer Raum für die Werkzeugaufnahme, die somit radial kleiner ausgebildet werden kann und daher für die Unterbringung innerhalb eines Handstückschafts prinzipiell geeignet ist. Hier hat es sich als besonders vorteilhaft erwiesen wenn für den kleineren und größeren Schaftdurchmesser die folgende Beziehung gilt:

$$d <= D/2$$

In anderen Worten ausgedrückt kann es vorgesehen sein, dass der Werkzeugschaftdurchmesser, an welchem die Geometrie angebracht/ausgeformt ist, die der Drehmomentmitnahme von der Werkzeugaufnahme dient, größer/gleich 2x des Durchmessers ist, durch welchen das Werkzeug axial gesichert wird. Dadurch wird der Aufbau einer halbautomatischen Kupplung/Werkzeugaufnahme (selbstständiges Verriegeln/manuelles Freigeben des Werkzeugs) mit stabilen, Drehmoment belastbaren Bauteilen und damit auch einer Einheitswerkzeuglänge unabhängig vom aktuell angeflanschten Hadstückschaft möglich. Erfindungsgemäß ist es vorgesehen, dass der Funktionsabschnitt "Drehmoment-Übertragung" mit einem Zweiflach ausgebildet ist, dessen zwei diametral voneinander abgewandte Angriffsebenen vorzugsweise keilförmig in Richtung Funktionsabschnitt "Axialverriegelung" aufeinander zulaufen. Dadurch kann der Werkzeugschaft im Wesentlichen verkantungsfrei in eine entsprechend geformte Werkzeugaufnahme axial eingeführt werden.

Eine weitere vorteilhafte optionale Maßnahme sieht vor, zwischen den beiden genannten Funktionsabschnitten ein Radialabsatz auszuformen. Dies erlaubt es wiederum, dass die Angriffsebenen durch eine entsprechend plane Verringerung des Schaftdurchmessers D im Funktionsabschnitt "Drehmoment-Übertragung" beispielsweise durch Fräsen, Schmieden, etc. hergestellt werden können. Somit ergibt sich durch den Zweiflach keine radiale Aufweitung des Werkzeugschafts.

Um den verklemmfreien Einsatz des Werkzeugs in die Werkzeugaufnahme zu verbessern, ist erfindungsgemäß ein Funktionsabschnitt "Eindrehhilfe" vorzugsweise als weiterer Bestandteil des Funktionsabschnitts "Drehmoment-Übertragung" oder unabhängig davon vorgesehen, der weiter vorzugsweise axial zwischen den Funktionsabschnitten "Drehmoment-Übertragung" und "Axialverriegelung" insbesondere im Bereich eines Radialabsatzes zwischen den Funktionsabschnitten "Drehmoment-Übertragung" und "Axialverriegelung" angeordnet ist. Gemäß der Erfindung hat der Funktionsabschnitt "Eindrehhilfe" mehrere, vorzugsweise vier, vier keilförmig ausgerichtete Abgleitebenen, von denen jeweils zwei Abgleitflächen zu beiden Längsseiten jeweils einer Angriffsebene in einem Winkel zu dieser ausgebildet sind und dabei die Längskanten der jeweiligen Angriffsebene zumindest abschnittsweise brechen. Auf diese Weise erfolgt das Eindrehen des Werkzeugs in die Werkzeugaufnahme erst mit in Eingriff kommen des Funktionsabschnitts "Drehmoment-Übertragung" mit der Werkzeugaufnahme und damit ohne zusätzliche Belastung des Funktionsabschnitts "Axialverriegelung".

Gemäß einem anderen Aspekt der vorliegenden Erfindung ist ein chirurgisches, Drehmoment übertragendes Instrument vorgesehen mit einer Werkzeugaufnahme für ein wahlweises Aufnehmen des drehbaren chirurgischen Werkzeugs gemäß der vorliegenden Erfindung bei gleichzeitiger axialen Sicherung des Werkzeugs in der Werkzeugaufnahme sowie Übertragung eines Drehmoments auf das Werkzeug. Dieses Instrument hat vorzugsweise ein (universelles) Handstück mit darin angeordnetem Drehmoment-Übertragungszug und/oder Antrieb und ein vorzugsweise auswählbarer (separater) Handstückschaft mit darin gelagerter Werkzeugaufnahme, der weiter vorzugsweise an das Handstück anflanschbar ist unter Kopplung der Werkzeugaufnahme an den Drehmoment-Übertragungszug oder Antrieb.

Die Erfindung wird nachstehend anhand eines bevorzugten Ausführungsbeispiels sowie unterschiedlicher Varianten unter Bezugnahme auf die begleitenden Figuren näher erläutert.

**Figurenbeschreibung**

Fig. 1 zeigt den Längsschnitt eines chirurgischen Instruments dieser Gattung (einschließlich Handstück, Handstückschaft und Werkzeug) wie es auch aus dem Stand der Technik bekannt ist und welches als Referenz zur besseren Darstellung der Erfindung dienen soll,

Fig. 2 zeigt in Vergrößerung den proximalen Schaft - Endabschnitt eines Werkzeugs für das chirurgische Instrument nach Fig. 1,

Fig. 3 zeigt den Längsschnitt eines chirurgischen Instruments/Systems einschließlich Handstück, Handstückschaft und Werkzeug gemäß einem bevorzugten Ausführungsbeispiel,

Fig. 4a, 4b zeigen in Vergrößerung den proximalen Schaft - Endabschnitt eines erfindungsgemäßen Werkzeugs für ein chirurgisches Instrument nach Fig. 3,

Fig. 5 zeigt eine vergrößerte Längsschnittdarstellung des erfindungsgemäßen Instruments im Bereich des Werkzeugschlosses (der Werkzeugaufnahme / Werkzeugschloss),

Fig. 6 zeigt eine vergrößerte Querschnittdarstellung der Werkzeugaufnahme entlang der Schnittlinie A-A gemäß der Fig. 5 mit bereits eingesetztem Werkzeug,

Fig. 7a und 7b zeigen jeweils eine Längsschnittdarstellung einer ersten und zweiten Variante der Lagerung des Werkzeugs bzw. der Werkzeugaufnahme in einer Gegenüberstellung,

Fig. 8 zeigt in Vergrößerung den proximalen Werkzeugschaft - Endabschnitt gemäß einer zu Fig. 4 alternativen Variante,

Fig. 9 zeigt ein Beispiel einer Kodierungsmöglichkeit von unterschiedlichen Werkzeugschäften zur verwechslungssicheren Verwendung in unterschiedlichen Werkzeugaufnahmen (unterschiedlichen Handstückschäften/Handstücken),

Fig. 10 stellt zwei Beispiele für eine korrekte und eine inkorrekte Auswahl eines Werkzeugs für jeweils ein Instrumentenhandstück/Handstückschaft (mit bestimmter Aufnahme) entsprechend der Kodierung nach Fig. 9 dar,

Fig. 11 zeigt den Längsschnitt des chirurgischen Instrumentenhandstücks (nach Fig. 1) im Bereich des Werkzeugschlosses (Werkzeugaufnahme/-kupplung) ohne Werkzeug,

Fig. 12 und 13 beschreiben (chronologisch) den Montagevorgang zur Bereitstellung des Werkzeugschlosses (Werkzeugaufnahme) gemäß der Fig. 12

Fig. 14 zeigt den Querschnitt einer Kleeblattkupplung zur (lösbaren) (Drehmoment - Übertragungs -)Verbindung zwischen dem Werkzeugschloss (Werkzeugaufnahme oder Torsionswelle) und einer Abtriebswelle innerhalb des chirurgischen Instrumentenhandstücks für das Wechseln des distalen Handstückschafts,

Fig. 15 zeigt ein männliches und weibliches Teil der Kleeblattkupplung gemäß der Fig. 14 und

Fig. 16 zeigt schrittweise den Einführvorgang eines erfindungsgemäßen Werkzeugs in eine erfindungsgemäße Werkzeugaufnahme.

Das erfindungsgemäße chirurgische Instrument bzw. Instrumentensystem bestehend aus einem austauschbaren (Dreh-) Werkzeug, einem (universellen) Instrumentenhandstück und einem ggf. austauschbaren Handstückschaft (einschließlich darin gelagerter Torsionswelle) beinhaltet im Wesentlichen vier Teilaspekte, welche im Rahmen dieser Offenbarung unabhängig oder in Kombination miteinander ausführbar sind und welche nachfolgend im Einzelnen beschrieben werden. Dazu zählen

- die erfindungsgemäße Gestaltung des proximalen Schaft - Endabschnitts des erfindungsgemäßen Werkzeugs des vorliegenden Instrumentensystems,
- die Schaffung einer Einstecksicherung in Form einer Werkzeugkodierung zur Vermeidung eines Werkzeug - Auswahl-/ Anwendungsfehlers,
- die Konstruktion des Werkzeugschlosses (oder auch Werkzeugaufnahme) innerhalb des Handstückschafts des Instrumentenhandstücks als Teil des Drehmomentübertragungszugs auf das Werkzeug (zur Kopplung von Werkzeug mit Torsionswelle innerhalb des Handstückschafts) sowie die Werkzeugschlosskonstruktion in seinem Bedienabschnitt und
- die Ausbildung einer Kupplung/drehmomentfesten Verbindung zwischen Werkzeugschloss (Werkzeugaufnahme) bzw. Torsionswelle innerhalb des Handstückschafts und einer Abtriebswelle innerhalb des Handstücks zur Ermöglichung der Auswechslung des distalen Handstückschafts (einschließlich des darin gelagerten Werkzeugschlosses und der Torsionswelle).

## Erfindungsgemäßes Werkzeug mit axial getrennter Drehmomentübertragung, Werkzeugeindrehung/Ausrichtung und axialer Verriegelung

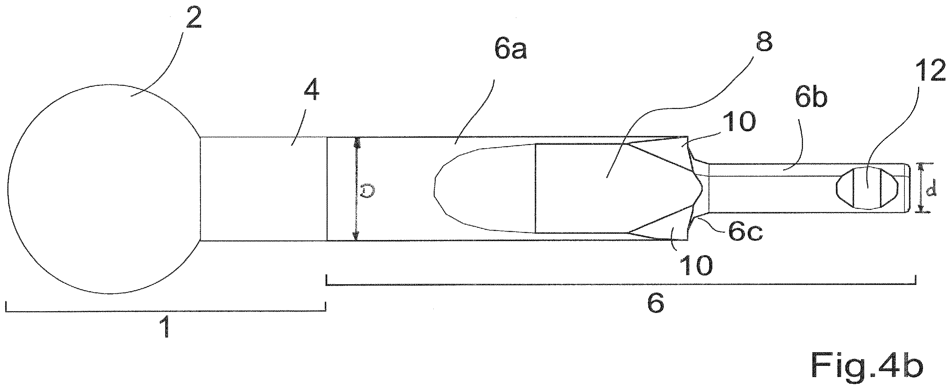

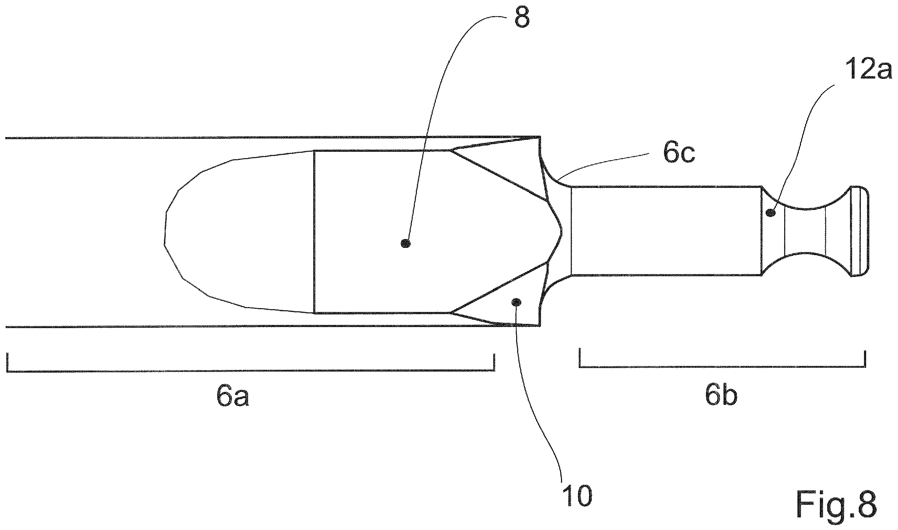

Gemäß der Fig. 4a, 4b und 8 besteht das erfindungsgemäße Werkzeug 1 grundsätzlich aus einem distalen (körperzugewandten) Eingriffssegment oder - abschnitt beispielsweise einem Bohr-, Fräs-, Schleif- oder Polierkopf 2, an den ein Werkzeugschaft 4 vorzugsweise stoffeinstückig (oder gelötet, geschweißt, gepresst, etc.) angesetzt ist, der sich in Richtung proximal (körperabgewandt) erstreckt. Dieser Werkzeugschaft 4 hat einen proximalen Endabschnitt 6 für das drehfeste Einsetzen des Werkzeugs 1 in eine Werkzeugaufnahme (Werkzeugschloss) eines chirurgischen Instrumentenhandstücks bzw. eines daran angeschlossenen Handstückschafts sowie für eine axiale Sicherung in der Werkzeugaufnahme.

Der Werkzeugschaft 1 ist hierfür in seinem proximalen Endabschnitt 6 in drei Funktionsbereiche unterteilt, welche axial voneinander (seriell) beabstandet sind und nachfolgend in chronologischer Reihenfolge ausgehend vom distalen Ende des Werkzeugschaft - Endabschnitts 6 (gemäß der Fig. 4a, 4b und 8 das linke Ende des proximalen Endabschnitts 6) beschrieben werden.

Wie aus den Fig. 4a, 4b und 8 zu entnehmen ist, besteht der gesamte Werkzeugschaft 4 gemäß der vorliegenden Erfindung zunächst aus einem distalen, im Wesentlichen unprofilierten Schaftabschnitt (schließt sich unmittelbar an das Eingriffssegment 2 an), sowie dem daran sich anschließenden proximalen Schaft-Endabschnitt 6, der wiederum in einen distalen Abschnitt 6a mit großem Schaftdurchmesser sowie einer Außenprofilierung und einem proximalen Abschnitt 6b mit kleinem Schaftdurchmesser seriell unterteilt ist. Das Durchmesserverhältnis zwischen großem und kleinem Schaftdurchmesser D:d innerhalb des proximalen Schaft-Endabschnitts 6 beträgt in etwa 2:1. D.h., der kleine Schaftdurchmesser d ist im Wesentlichen kleiner/gleich der Hälfte des großen Schaftdurchmessers D. Konkreter ausgedrückt soll gelten: d <= 0,6D. Dabei verjüngt sich der große Schaftdurchmesser D nicht kontinuierlich zum kleinen Schaftdurchmesser d sondern es bildet sich ein Radialabsatz 6c zwischen beiden Schaftabschnitten 6a, 6b unterschiedlichen Durchmessers ggf. mit einem geringen Innenradius zur Verminderung einer Kerbwirkung.

Im Bereich des Radialabsatzes 6c ist der großdurchmessrige Schaftabschnitt 6a gemäß der Fig. 4a mit zwei diametral sich gegenüberliegenden Angriffsflächen 8 oder -ebenen ausgebildet (sogenannter Zweiflach), die sich keilförmig in Richtung hin zum Radialabsatz 6c annähern und zur Einleitung eines Drehmoments in den Werkzeugschaft 4 dienen. Diese Angriffsebenen 8 können insbesondere durch Anschleifen/-fräsen oder durch Pressen/Schmieden des zunächst unprofilierten, runden Werkzeugschafts 4 gebildet sein. An den axialen Seitenkanten jeder Angriffsfläche 8 sind (im Bereich des Radialabsatzes 6c) zusätzliche Abgleitflächen oder - ebenen 10 ausgebildet (vorzugsweise hergestellt wie die Angriffsflächen 8), die jeweils in einem Winkel zur zugehörigen Angriffsfläche 8 ausgerichtet sind und ausgehend von einem axialen Mittenbereich jeder Angriffsebene-Seitenkante in Richtung hin zum Radialabsatz 6c keilförmig zulaufen. Dadurch entsteht im Bereich des Radialabsatzes 6c ein Schaftprofil mit sechs Flächen bestehend aus den zwei diametral gegenüberliegenden Angriffsebenen 8 (Zweiflach) sowie in Umfangsrichtung zu beiden Seiten jeder Angriffsebene 8 jeweils einer Abgleit- oder Eindrehebene 10, welche die entsprechende Seitenkante der jeweiligen Angriffsfläche 8 im Bereich des Radialabsatzes 6c bricht und damit die Breite der jeweiligen Angriffsebene 8 in Richtung hin zum Radialabsatz 6c zunehmend verringert.

Am proximalen Ende des kleindurchmessrigen Schaft-Endabschnitts 6b sind gemäß der Fig. 4a und 4b zwei diametral am Schaftumfang angeordnete Kerben oder Taschen 12 ausgeformt (vorzugsweise eingefräst), wodurch sich axial wirkende Hinterschneidungen an der Schaftoberfläche bilden. Alternativ zu diesen Kerben 12 ist es aber gemäß der Fig. 8 auch möglich, am proximalen Ende des kleindurchmessrigen Schaft-Endabschnitts 6c eine umlaufende Nut 12a auszudrehen, deren Nutentiefe im Wesentlichen der Kerbentiefe gemäß der Fig. 4a, 4b entspricht. Diese Kerben 12 oder Umfangsnut 12a dienen zur axialen Verriegelung des Werkzeugschafts 4 innerhalb einer Werkzeugaufnahme, wie sie nachfolgend noch beschrieben wird.

Durch die vorstehend beschriebene Schaftkonstruktion insbesondere im profilierten Werkzeugschaft-Endabschnitt 6 lassen sich gegenüber dem Stand der Technik gemäß der Fig. 1 und 2 einige Vorteile erzielen, welche zur Steigerung des maximal übertragbaren Drehmoments von der Torsionswelle innerhalb des Handstückschafts auf das Werkzeug 1 beitragen:

- Durch die prinzipielle Trennung von axialer Sicherung/Verriegelung und Drehmomentmitnahme (mit dazwischen angeordneter Eindrehhilfe) in zwei (ggf. drei) axial beabstandete Schaftabschnitte können diese Funktionsabschnitte unabhängig voneinander optimiert werden.

- Entscheidend dabei ist, dass der Funktionsabschnitt "Verriegelung" 6b bezüglich des Funktionsabschnitts "Drehmomentmitnahme" 6a proximal angeordnet ist. Dies ermöglicht es, den Verriegelungsabschnitt 6b, der nicht auf Torsion/Drehmoment belastet wird, gegenüber dem Drehmomentmitnahmeabschnitt 6a kleindurchmessrig auszugestalten und dadurch den Radialabsatz 6c zu bilden.

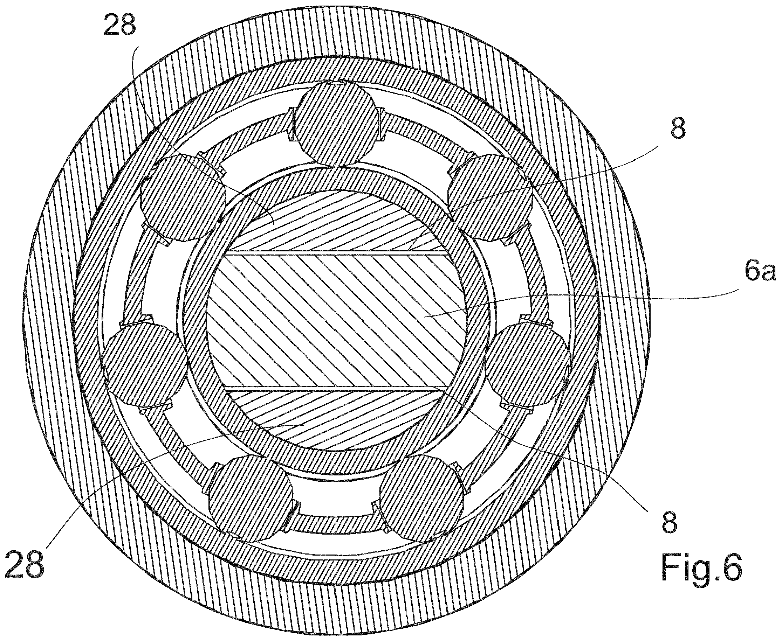

- Der Radialabsatz 6c wiederum erlaubt es, distal zum kleindurchmessrigen Verriegelungsabschnitt 6b zwei Angriffs- oder Drehmomentübertragungsebenen 8 mit größerer axialer Länge auszuformen (abzufräsen), um dadurch deren jeweilige Fläche gegenüber dem Stand der Technik zu vergrößern. Durch den Radialabsatz 6c kann zudem (wie insbesondere in der Fig. 6 dargestellt ist) in dessen Bereich so viel Schaftmaterial zur Ausbildung der Angriffsebenen 8 abgetragen werden, dass sich der verbleibende Schaftdurchmesser (im Radialabsatzbereich) zwischen den beiden (keilförmigen) Ebenen 8 nahezu halbiert. Damit nähert sich gemäß der Fig. 6 der zur Drehmomentübertragung nutzbare Durchmesser Dm dem großen Schaftdurchmesser D an. Wird somit der so ausgebildete Zweiflach in einen Axialspalt einer Mitnahmewelle geschoben (wird nachfolgend noch beschrieben), ergibt sich gemäß der Fig. 6 wieder ein Vollkreis mit optimalem Hebelverhältnis zur Drehmomentübertragung.

- Bisher wurde die axiale Verriegelung gemäß der Fig. 1 und 2 zwischen dem Funktionsabschnitt "Drehmomentmitnahme" und dem WerkzeugEingriffssegment angeordnet, wodurch sich die maximal mögliche axiale Ausdehnung des Funktionsabschnitts "Drehmomentmitnahme" beschränkt. Durch die dadurch sich ergebende steilere Keilform der beiden Angriffsebenen wirken große axiale Kräfte auf die axiale Verriegelung. Zudem wird der im Drehmomentfluss angeordnete Verriegelungsabschnitt materiell geschwächt. Nunmehr ist es vorgesehen, die axiale Verriegelung 6b proximal zur Drehmomentmitnahme 6a (also nicht zwischen Drehmomentmitnahme und Eingriffssegment) außerhalb des Drehmomentflusses zu platzieren. Damit kann die Keilform der beiden Angriffsebenen 8 insgesamt abgeflacht werden (unter größerer axialer Ausdehnung), wodurch sich axiale Kräfte bei der Drehmomentübertragung verringern. Somit kann der benötige (radiale) Bauraum für die axiale Verriegelung 6b verkleinert werden (kleiner Werkzeugschaftdurchmesser d möglich).

- Schließlich wird durch die Ausbildung des Radialabsatzes 6c zwischen den Funktionsabschnitten "Drehmomentübertragung" 6a und "axiale Verriegelung" 6b die Möglichkeit der Anordnung zusätzlicher Abgleitebenen 10 als Eindrehhilfe im Funktionsabschnitt "Drehmomentübertragung" 6a gegeben. Diese Abgleitebenen 10 sind jeweils zu beiden axialen Seiten der beiden Angriffsebenen 8 ebenfalls keilförmig ausgebildet und brechen dabei die Seitenkanten der Angriffsebenen 8 im Bereich des Radialabsatzes 6c - d.h. sie sind in einem Winkel zur jeweiligen Angriffsebene 8 ausgerichtet. Diese Abgleitebenen 10 dienen dazu, den Werkzeugschaft 4 bei Einführen in eine Werkzeugaufnahme des Handstücks in Umfangsrichtung auszurichten, derart, dass die beiden Angriffsebenen 8 korrekt in die Werkzeugaufnahme geleitet werden.


**Werkzeug mit Werkzeugkodierung**

Wie vorstehend bereits ausgeführt wurde, besteht ein Merkmal der vorliegenden Erfindung darin, den Funktionsabschnitt "axiale Verriegelung" 6b proximal zum Funktionsabschnitt "Drehmomentübertragung" 6a anzuordnen. Darüber hinaus weist der erfindungsgemäße Werkzeugschaft 4 auch noch weitere Merkmale gemäß der vorstehenden Beschreibung auf, wobei diese jedoch für den nachfolgenden Aspekt "Werkzeugkodierung" nur optional sind.

Grundsätzlich existiert ein Anwenderwunsch, Behandlungsfehler insbesondere infolge falscher chirurgischer Werkzeuge zu minimieren oder auszuschließen. Dies lässt sich beispielsweise durch optische Kennungen an den einzelnen Werkzeugen bewerkstelligen, wobei jedoch in diesem Fall der Faktor "Mensch" als Fehlerquelle nicht ausgeschlossen werden kann. D.h., in der Praxis können optische Kennungen übersehen oder missverstanden/verwechselt werden, sodass es bei der Auswahl eines bestimmten Werkzeugs zu Fehlern kommen kann, die erst bei deren Einsatz ggf. zu spät erkannt werden. Diese Fehlerquelle ist umso bedeutender, je mehr verschiedene Werkzeuge im Rahmen eines Instruments/Instrumentensystems einem universellen Handstück zugeordnet werden können. In diesem Fall ist es daher vorteilhaft und wünschenswert, wenn für bestimmte chirurgische Einsatzzwecke in Abhängigkeit eines bestimmten, an das Universalhandstück angeschlossenen Handstückschafts (mit innen gelagerter Torsionswelle) nur eine begrenzte Zahl an Werkzeugen eingesetzt werden kann.

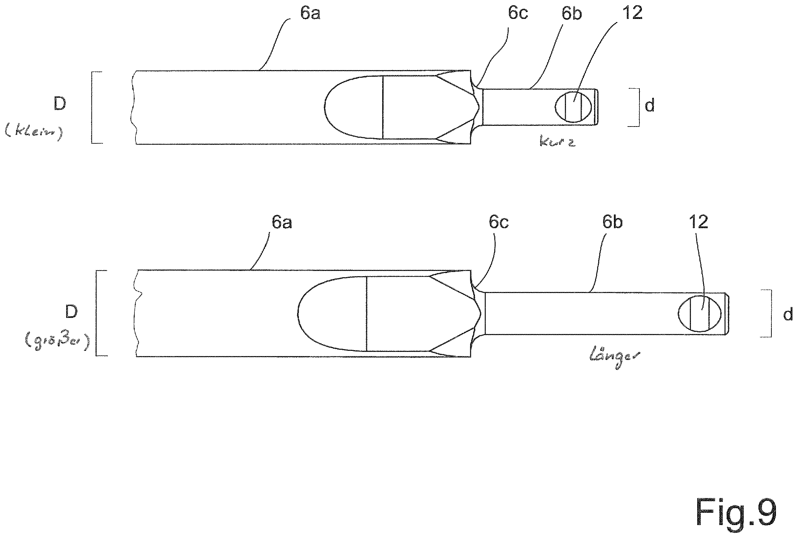

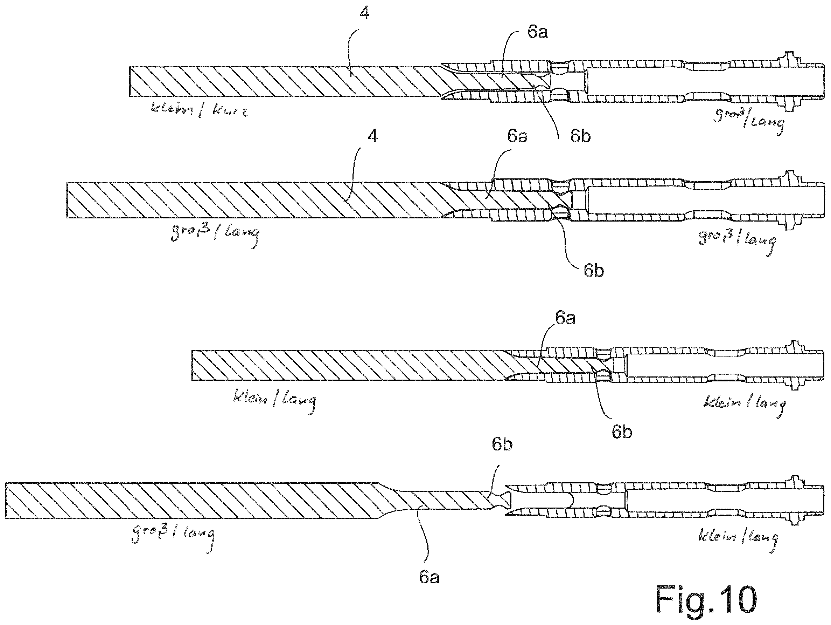

In den Fig. 9 und 10 ist eine vorteilhafte Variante einer Werkzeugkodierung dargestellt, durch die eine Falschauswahl eines Werkzeugs vermeidbar ist.

Die drehmomentfreie Anordnung des Funktionsabschnitts "axiale Verriegelung" 6b proximal zum Funktionsabschnitt "Drehmomentübertragung" 6a bietet die grundsätzlichen (optionalen) Möglichkeiten, die axiale Länge und/oder den (kleindurchmessrigen) Schaftdurchmesser d dieses Funktionsabschnitts 6b zu verändern, ohne dass hierdurch der Funktionsabschnitt "Drehmomentübertragung" 6a (nachteilig) beeinflusst wird. D.h. es wird hierdurch möglich, wenigstens zwei (oder mehrere) unterschiedliche axiale Abschnittslängen (d.h. axialer Abstand zwischen Radialabsatz 6c und radiale Tasche/Umfangsnut 12/12a bzw. axial wirkende Hinterschneidung) und/oder wenigstens zwei (oder mehrere) unterschiedliche (kleindurchmessrige) Schaftdurchmesser d vorzusehen (bzw. zu kombinieren), die nur mit entsprechend dimensionierten Werkzeugaufnahmen funktionell zusammenwirken können.

Beispielsweise sind in der Fig. 9 die beiden Kombinationen "kurzer Verriegelungsabschnitt" mit "kleinerem Schaftdurchmesser" und "langer Verriegelungsabschnitt" mit "größerem Schaftdurchmesser" bezüglich des Funktionsabschnitts "Verriegelung" 6b gezeigt. Demzufolge wird die Werkzeugaufnahme (welche nachfolgend noch detailliert beschrieben wird) gemäß der Fig. 10 prinzipiell so ausgebildet, dass zwar der kleinere Schaftdurchmesser auch in die Aufnahme für den größeren Schaftdurchmesser für eine Drehmomentübertragung einführbar ist, jedoch keine axiale Verriegelung stattfindet und daher das Werkzeug 1 bei Überprüfung des korrekten Werkzeugsitzes wieder herausgezogen werden kann (obere Darstellung). Wird indessen in diese Werkzeugaufnahme der größere Schaftdurchmesser eingeführt, findet eine axiale Verriegelung statt (zweite Darstellung von oben). Im Gegenzug erlaubt eine Aufnahme für den kleineren Schaftdurchmesser erst gar kein Einführen des größeren Schaftdurchmessers (untere Darstellung), wohingegen der kleinere Schaftdurchmesser eingeführt und axial verriegelt werden kann (zweite Darstellung von unten).

An dieser Stelle sei darauf hingewiesen, dass die Länge und der Schaftdurchmesser des Funktionsabschnitts "axiale Verriegelung" 6b nur zwei Kodierparameter darstellen, die besonders einfach erkennbar sind, die jedoch auch durch andere Parameter ersetzt oder ergänzt werden können. Beispielsweise kann die Umfangsposition der Taschen 12 bezüglich der beiden Angriffsebenen 8 dazu dienen, ein Verriegeln nur bei der richtigen vorbestimmten Relativposition (bei entsprechend korrekter Ausrichtung der Angriffsebenen 8 zur Werkzeugaufnahme) zu ermöglichen. Auch die Form der Taschen 12 ist veränderbar, derart, dass nur kompatible Formen auf Seiten der Aufnahme eine sichere axiale Verriegelung ergeben. Schließlich kann der Abschnitt "axiale Verriegelung" 6b mit einer zusätzlichen Form ausgestaltet sein (nicht dargestellt), die nach dem "Schlüssel-Schlüsselloch-Prinzip" mit einer entsprechenden Form in der Werkzeugaufnahme zusammenwirkt, um ein Einführen der Werkzeugschafts 4 zu erlauben (z.B. Nut-Feder-Anordnung).

**Handstückschaft mit Werkzeugaufnahme (oder auch Werzeugschloss)**

Eine in einem Handstückschaft unterzubringende Werkzeugaufnahme insbesondere für ein (Einheits-) Werkzeug gemäß dem vorstehend beschriebenen Aspekt der Erfindung hat mehrere Anforderungen zu erfüllen, die im Wesentlichen die Folgenden umfassen:

- Geringe radiale Abmessungen zur Ermöglichung von deren Unterbringung in einem bekanntlich engen Handstückschaft.
- Übertragung eines ausreichenden Arbeitsdrehmoments auf das Werkzeug.
- Ergonomisch günstige und einfache manuelle Betätigung zumindest zur Freigabe des darin eingesetzten Werkzeugs und vorzugsweise automatisches Verriegeln des Werkzeugs (halbautomatische Werkzeugaufnahme).
- Sicherung von Werkzeugaufnahme und Werkzeug im Betrieb gegen selbsttätige Demontage (etwa bei Vibrationen, Stößen und/oder Schlägen) zur Erhöhung der Instrumentenzuverlässigkeit.
- Einfaches und zerstörungsfreies Montieren und Demontieren der Aufnahme beispielsweise zu Reinigungs- oder Wartungszwecken.

Der Zweck einer solchen Aufnahme innerhalb des Handstückschafts besteht dabei grundsätzlich darin, die Werkzeugaufnahme beliebig (möglichst) weit nach distal zu verlagern und damit den Werkzeugschaft auf eine bezüglich der zu erwartenden Biegekräfte während des Werkzeugeinsatzes optimale (Einheits-) Länge zu beschränken. Damit kann ein solches (Einheits-) Werkzeug für unterschiedliche Schaftlängen und Schaftformen vorgesehen sein, wobei die Schaftstrecke zwischen Handstück und Werkzeugaufnahme durch eine im Handstückschaft gelagerte ggf. biegeflexible oder starre Torsionswelle überbrückt wird.

Die in der Fig. 1 schematisch dargestellte, bekannte Werkzeugaufnahme hat zwar hinsichtlich ihrer räumlichen (insbesondere radialen) Abmessungen das Potential, innerhalb eines per se bekannten Handstückschafts bekannter Bauform verbaut zu werden. Indessen stellen insbesondere die Querstifte zur Verbindung des Aufnahmerohrs mit der Torsionswelle sowie zur drehfesten Kopplung des Aufnahmerohrs mit dem darin gelagerten, auf das Werkzeug einwirkenden Drehmoment-Übertragungsbolzen jeweils eine Schwachstelle im Drehmoment-Übertragungszug dar, wie dies eingangs bereits beschrieben wurde.

Wie die Fig. 1 im Detail nämlich zeigt, ist der innen liegende Drehmoment-Übertragungsbolzen mittels des einen (dünnen) Querstifts, der in einer darin ausgebildeten Längs-Querbohrung lagert, zumindest mit dem äußeren Aufnahmerohr gekoppelt. Ein für alle Anwendungszwecke geeignetes maximales Drehmoment ist mit einem derartigen (dünnen) Querstift nicht sicher übertragbar. Die Bohrungen für den Querstift schwächen zusätzlich die ohnehin sehr kleinen zu verbindenden Bauteile, nämlich Aufnahmerohr und Bolzen. Außerdem ist, wie dies vorstehend bereits ebenfalls geschildert wurde, ein weiterer Querstift zur Kopplung des Aufnahmerohrs mit der Eingangs- bzw. Torsionswelle vorgesehen, der die gleichen Probleme verursacht. Unabhängig davon ist das Verbinden von demnach drei Bauteilen mittels der genannten Querstifte fertigungs- und montagetechnisch besonders bei kleinen Dimensionen wie bei den gattungsgemäßen Handstückschäften einschlägig bekannter Bauart sehr schwierig und zeitraubend. Daher ist es wünschenswert, eine Werkzeugaufnahme insbesondere für ein Werkzeug mit einem vorstehend beschriebenen Aufbau bereit zu stellen, welche diese Probleme löst.

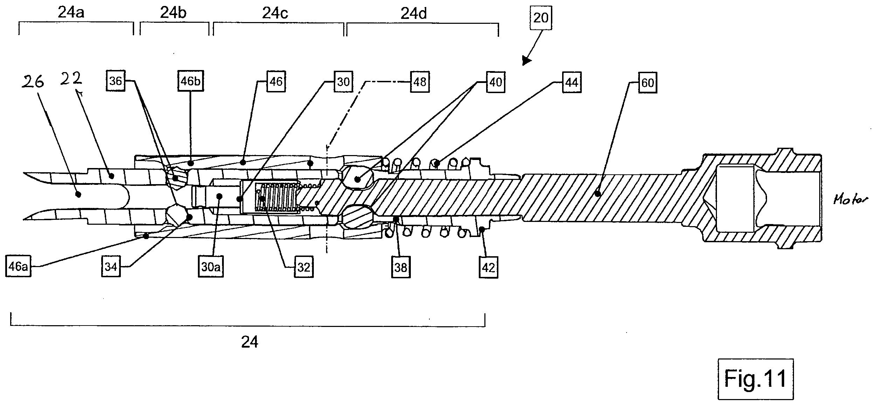

Die Fig. 11 zeigt ein bevorzugtes Ausführungsbeispiel einer solchen Werkzeugaufnahme 20, deren Bauteile nachfolgend im Einzelnen sowie in deren Zusammenwirken mit dem eingangs beschriebenen Werkzeug 1 verläutert werden.

Zunächst hat die Werkzeugaufnahme oder Werkzeugschloss 20 gemäß dem bevorzugten Ausführungsbeispiel der vorliegenden Offenbarung ein radial inneres Werkzeug-Aufnahmerohr (nachfolgend als Mitnehmerwelle bezeichnet) 22 mit einem an ihrem distalen Ende längsgeschlitzten (schnabelförmigen) distalen Drehmoment-Übertragungs- und Verriegelungsabschnitt 24, der in seinem geschlitzten Drehmoment-Übertragungsbereich 24a (siehe auch die Fig. 6) einen an den großdurchmessrigen Werkzeugschaftabschnitt 6a angepassten Außendurchmesser und in seinem daran sich anschließenden Verriegelungsbereich 24b einen an den kleindurchmessrigen Werkzeugschaftabschnitt 6b angepassten Innendurchmesser hat. Der Längsschlitz 26 bildet dabei ein Schlitzweite, in die der Werkzeugschaft 1 im Bereich der beiden keilförmigen Angriffsebenen 8 eingeschoben werden kann (siehe Fig. 6), sodass die werkzeugseitigen Angriffsebenen 8 an den dadurch entstandenen schnabelförmigen Axialvorsprüngen 28 des Drehmoment-Übertragungsbereichs 24a flächig anliegen und dabei gemeinsam ein geschlossenes Voll-Rundprofil bilden (siehe Fig. 6).

An den Verriegelungsbereich 24b schließt sich proximal ein zylindrischer Bolzenaufnahmeabschnitt 24c mit gegenüber dem Verriegelungsbereich 24b größerem Innendurchmesser (der darin gelagerte Bolzen 30 wird nachfolgend als Nachfahrelement bezeichnet) unter Ausbildung eines inneren Radialabsatzes an, der als Axialanschlag für das Nachfahrelement 30 in Richtung distal dient. Das Nachfahrelement 30 hat hierfür einen distalen Abschnitt 30a mit einem Außendurchmesser entsprechend dem kleindurchmessrigen Werkzeugschaftabschnitt "Verriegelung" 6b, der somit in den Verriegelungsbereich 24b der Mitnehmerwelle 22 eingefahren werden kann sowie einen proximalen Abschnitt mit größerem Außendurchmesser 30b, an dem das Nachfahrelement 30 gleitend in der Mitnehmerwelle 22 geführt ist. Zwischen den beiden Abschnitten 30a, 30b des Nachfahrelements 30 bildet sich ebenfalls ein äußerer Ringabsatz aus, der mit dem inneren Ringabsatz der Mitnehmerwelle 22 in Richtung distal zusammenwirkt.

Schließlich ist in dem Aufnahmeabschnitt 24c für das Nachfahrelement 30 eine Nachfahrfeder 32 angeordnet, welche das Nachfahrelement 30 in Richtung distal vorspannt und so gegen den inneren Ringabsatz in der Mitnehmerwelle 22 drückt. In dieser Position ist der kleiner durchmessrige distale Abschnitt 30a des Nachfahrelements 30 vollständig in den Verriegelungsbereich 24b der Mitnehmerwelle 22 eingefahren.

An dieser Stelle sei erwähnt, dass die Mitnehmerwelle 22 in deren Verriegelungsbereich 24b eine Anzahl (mindestens eine) von gleichmäßig umfangsbeabstandeten radialen Durchgangsbohrungen 34 hat, die zur Aufnahme von Verriegelungskugeln 36 für das eingesetzte Werkzeug 1 dienen, wie dies nachfolgend noch beschrieben wird.

In proximaler Verlängerung des Aufnahmeabschnitts 24c für das Nachfahrelement 30 bildet die Mitnehmerwelle 22 einen Kopplungs-/Einsteckabschnitt 24d für eine Antriebs-/Torsionswelle 60, welche in einem in der Fig. 11 nicht dargestellten Handstückschaft (siehe beispielsweise Fig. 3) eines (universellen) Handstücks drehbar gelagert ist.

In diesem Einsteckabschnitt 24d hat die Mitnehmerwelle 22 ebenfalls eine Anzahl (wenigstens eine) gleichmäßig umfangsbeabstandeter sowie auf einer Kreisebene liegender Radialöffnungen oder Durchbrüche 38 von in etwa ovalem Querschnitt, der sich jeweils in Axialrichtung der Mitnehmerwelle 22 ausdehnt. Diese Radialöffnungen 38 dienen zur Aufnahme von vorzugsweise ovalen Wälzkörpern 40 (nachfolgend als Mitnahmeelemente bezeichnet), über welche die Mitnehmerwelle 22 dreh- und axialfest mit der eingesteckten Torsionswelle 60 gekoppelt ist, was nachfolgend noch näher beschrieben wird. Dabei sei noch erwähnt, dass anstelle ovaler (zylindrischer) Wälzkörper mit abgerundeten Stirnseiten auch Kugeln verwendet werden können.

Am proximalen Ende des Einsteckabschnitts 24d hat die Mitnehmerwelle 22 ferner einen umlaufenden Radialvorsprung 42, der als Federsitz einer äußeren Schließfeder 44 dient.

Um die Mitnehmerwelle 22 herum ist eine Schließhülse 46 dreh- und axial verschiebbar gelagert. Diese hat einen distalen Kugel-Freigabebereich 46a mit großem Innenradius und einen daran proximal sich anschließenden Kugel-Sperrbereich 46b mit kleinem Innenradius, der auch gleitend an der Außenseite der Mitnehmerwelle 22 geführt ist.

In einem proximalen Endabschnitt der Schließhülse 46 sind eine Anzahl (vorzugsweise zwei) von radialen Durchgangsöffnungen 48 mit längsovalem (oder rundem) Querschnitt ausgeformt, die zur Befüllung der in der Mitnehmerwelle 22 vorgesehenen Durchbrüche 38 mit den ovalen/tonnenförmigen, abgerundeten Wälzkörpern 40 dienen. Jede dieser ovalen (größere Länge als Breite) Durchgangsöffnungen 48 der Schließhülse 46 ist am Innenumfang der Schließhülse 46 zu einer Aufnahmetasche verlängert, sodass die Schließhülse 46 die bereits eingelegten Wälzkörper/Mitnahmeelemente 40 axial überfahren und gegen ein Herausfallen verriegeln kann. Gleichzeitig sind die Aufnahmetaschen so geformt, dass die Schließhülse 46 bezüglich der Mitnehmerwelle 22 um einen bestimmten Winkel gedreht werden kann, sodass auch bei einem Zurückfahren der Schließhülse 46 in eine axiale Freigabeposition die Wälzkörper 40 (und auch die Verriegelungskugeln 36 gemäß nachfolgender Beschreibung) nicht mehr herausfallen können.

Schließlich ist axial zwischen der Schließhülse 46 und dem äußeren Radialvorsprung 42 der Mitnehmerwelle 22 die Schließfeder 44 angeordnet, welche die Schließhülse 46 in Richtung distal in eine axiale Verriegelungsposition drückt.

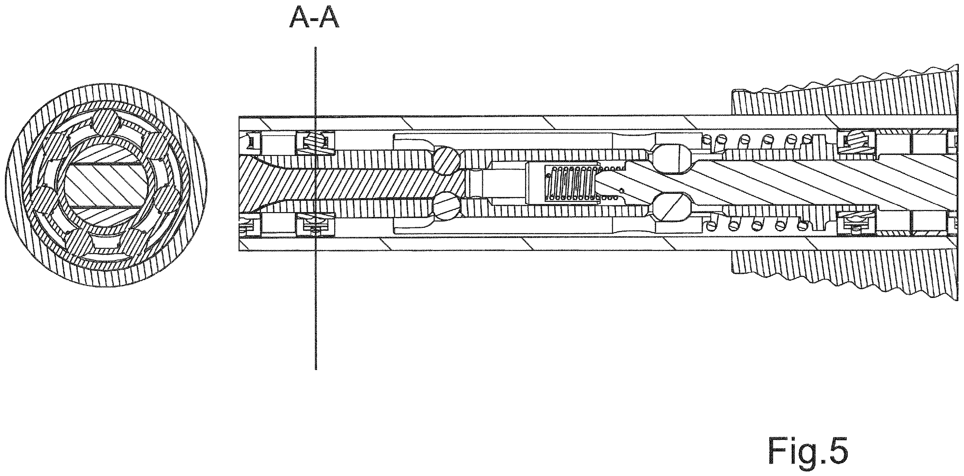

Die Montage und Funktionsweise der Werkzeugaufnahme 20 wird nachfolgend anhand der Fig. 11 bis 13 in Verbindung mit der Fig. 5 näher erläutert.

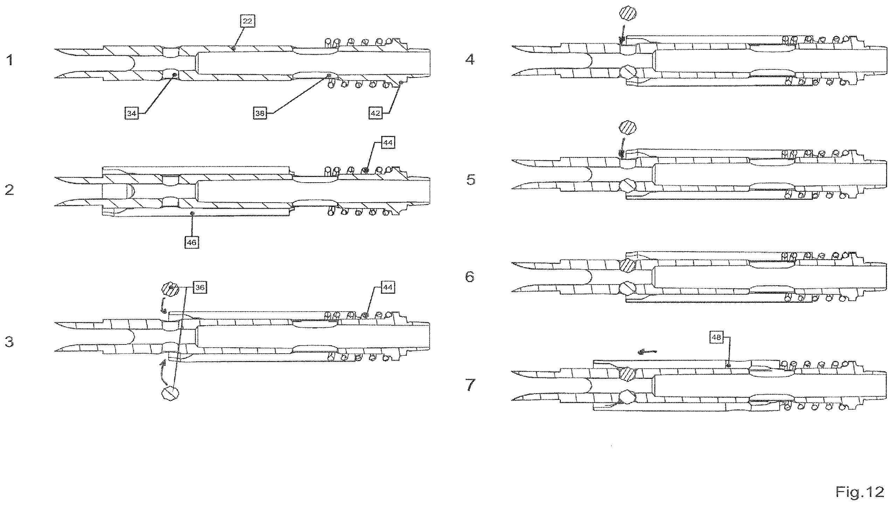

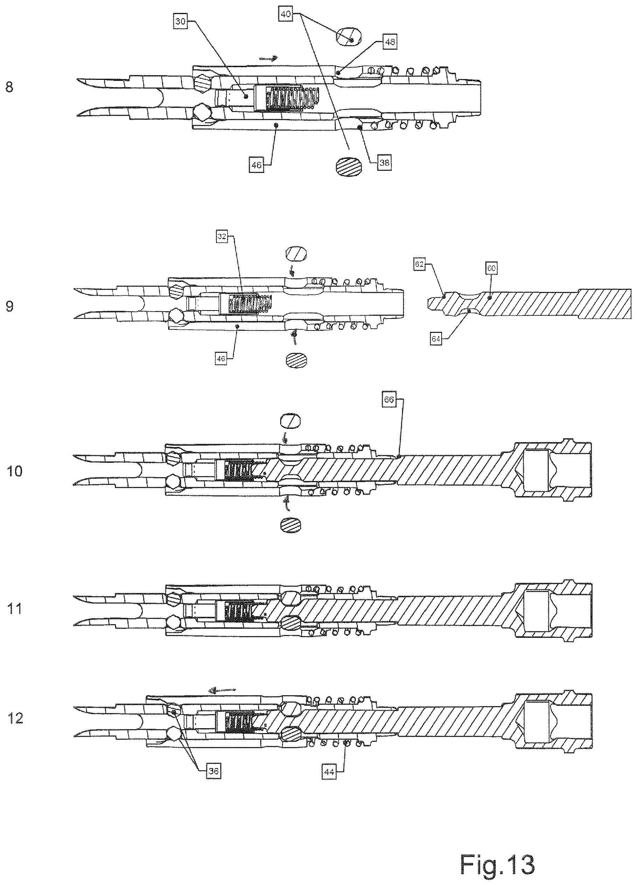

Gemäß der Fig. 12 und 13 wird für eine Montage der Werkzeugaufnahme 20 an einer Torsionswelle 60 zunächst die äußere Schließfeder 44 über die Mitnehmerwelle 22 gestreift und anschließend die Schließhülse 46 auf die Mitnehmerwelle 22 aus der Richtung distal aufgesteckt, sodass die äußere Schließfeder 44 zwischen der Schließhülse 46 und dem äußeren Radialvorsprung 42 an der Mitnehmerwelle 22 zu liegen kommt (siehe Abbildungen 1 und 2 der Fig. 12).

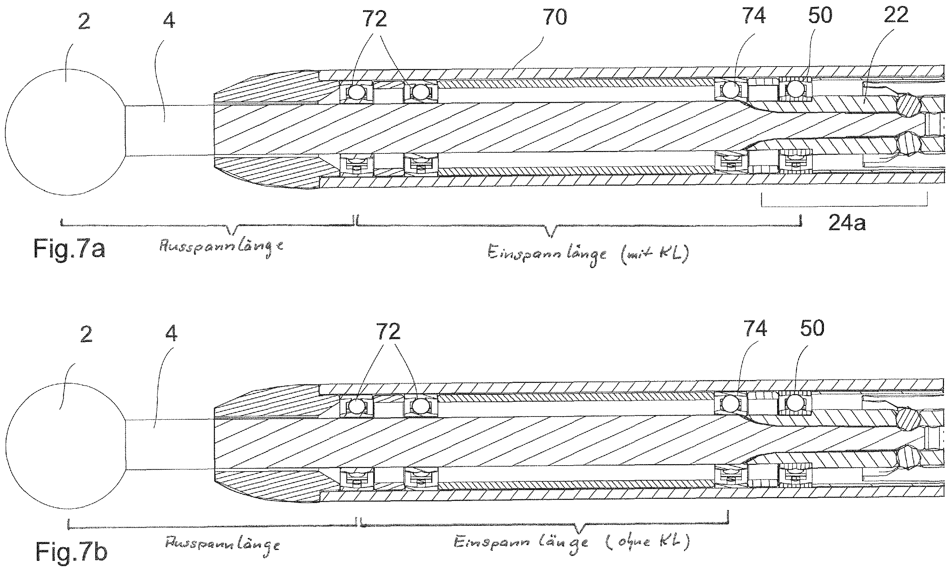

Anschließend wird die Schließhülse 46 gegen die äußere Schließfeder 44 in ihre axiale Befüll- bzw. Freigabeposition gedrückt, wodurch die Durchgangsbohrungen 34 im Verriegelungsbereich 24b der Mitnehmerwelle 22 freigelegt werden. Jetzt können in diese Durchgangsbohrungen 34 die Verriegelungskugeln 36 über eine Montagenut an der Innenseite der Schließhülse 46 eingesetzt werden, die radial nach Innen vorragen (siehe Abbildungen 3 bis 6 der Fig. 12). Abschließend kann die Schließhülse 46 freigegeben werden, wodurch diese durch die äußere Schließfeder 44 axial in Kugelsperrposition verschoben wird, in der die Schließhülse 46 die Verriegelungskugeln 36 überfährt und somit gegen ein radiales Herausfallen sichert. Die Verriegelungskugeln 36 dienen dabei gleichzeitig als Axialanschlag für die Schließhülse 46, die hierfür an ihrem distalen Innenumfang einen kleinen Innen-Radialabsatz hat, der in Sperr-/Verriegelungsposition der Schließhülse 46 axial an den Verriegelungskugeln 36 anliegt (siehe Abbildung 7 der Fig. 12). Damit ist die Vormontage der Werkzeugaufnahme 20 abgeschlossen.

In der Fig. 13 ist nunmehr die Montage der Werkzeugaufnahme 20 an einer Torsionswelle 60 dargestellt.

Zunächst wird in die Mitnehmerwelle 22 aus der Richtung proximal das Nachfahrelement 30 und daraufhin die innere Nachfahrfeder 32 eingesetzt, wobei der distale Abschnitt 30a des Nachfahrelements 30 an den Verriegelungskugeln 36 axial anliegt. Anschließend wird die Torsionswelle 60 in die Mitnehmerwelle 22 aus der Richtung proximal eingesteckt. Die Torsionswelle 60 bildet an ihrem distalen Ende einen radialen Absatz 62 als Federsitz für die bereits eingelegte innere Nachfahrfeder 32. Des Weiteren hat die Torsionswelle 60 an ihrem distalen Endabschnitt eine Anzahl von gleichmäßig umfangsbeabstandeten Außentaschen 64 zur Aufnahme der Mitnahmeelemente (ovale Wälzkörper) 40. Schließlich bildet die Torsionswelle 60 umfangsseitig optional einen Wellenabsatz 66 als Axialanschlag für die Mitnehmerwelle 22.

Sobald die Mitnehmerwelle 22 am optionalen Axialanschlag 66 der Torsionswelle 60 anliegt, überlappen sich die radialen Außentaschen 64 der Torsionswelle 60 genau mit den proximalen Durchbrüchen 38 der Mitnehmerwelle 22 sowie den Befüllöffnungen 48 der in die axiale Befüll-/Freigabeposition geschobenen Schließhülse 46 (siehe die Abbildungen 8 bis 10 der Fig. 13). Jetzt können die ovalen Mitnahmeelemente 40 über die Befüllöffnungen 48 der Schließhülse 46 in die Durchbrüche 38 der Mitnehmerwelle 22 sowie die Außentaschen 64 der Torsionswelle 60 eingesetzt werden (siehe Abbildung 11 der Fig. 13). Abschließend wird die Schließhülse 46 freigegeben, die selbsttätig durch die Schließfeder 44 in die Verriegelungsposition axial in Richtung distal verschoben wird, in welcher die Verriegelungskugeln 36 wie auch die Mitnahmeelemente 40 von der Schließhülse 46 überfahren und somit gegen ein radiales Herausfallen gesichert werden. Abschließend wird die Schließhülse 46 um einen bestimmten Winkel bezüglich der Mitnehmerwelle 22 gedreht. Dadurch wird verhindert, dass auch bei einem erneuten Zurückziehen der Schließhülse 46 in die Kugel-Freigabeposition während eines normalen Betriebs die Kugeln 36 und vorzugsweise die Mitnahmeelemente 40 über die Befüllöffnungen 48 der Schließhülse 46 unbeabsichtigt herausfallen können. D.h. die Axialposition der Schließhülse 46 für ein Befüllen mit den Mitnahmeelementen 40 sowie für ein radiales Freigeben der Kugeln 36 beim Einstecken eines Werkzeugs 1 ist vorzugsweise gleich. Die Winkelposition der Schließhülse 46 bezüglich der Mitnehmerwelle 22 in der Befüllposition ist jedoch zur Winkelposition in der Freigabeposition unterschiedlich.

Damit ist der Montagevorgang der Werkzeugaufnahme 20 an der Torsionswelle 60 abgeschlossen.

Wie die vorstehende Beschreibung des Montagevorgangs verdeutlicht, sind radial äußere Mitnahmeelemente 40 vorzugsweise in Form von ovalen Wälzkörpern für eine Drehmomentübertragung von der Torsionswelle 60 auf die Mitnehmerwelle 22 vorgesehen. Diese Elemente besitzen folglich eine große wirksame Kraftangriffsfläche und können daher erhebliche Drehmomente übertragen, ohne abzuscheren. Gleichzeitig dienen die Mitnahmeelemente zur Axialsicherung der Werkzeugaufnahme auf der Torsionswelle. Durch die radial äußere Positionierung wird zudem ein maximaler Hebel zur Drehmomentübertragung erreicht.

Das Drehmoment wird nicht wie im genannten Stand der Technik über das Nachfahrelement (Bolzen) 30 sondern unmittelbar über die Mitnehmerwelle 22 auf den Werkzeugschaft 4 übertragen. Dadurch reduzieren sich die in den Drehmoment-Übertragungszug eingebundenen Bauteile, wodurch die Montage insgesamt vereinfacht wird.

Die Funktionsweise der Werkzeugaufnahme 20 wird nachstehend anhand der Fig. 5, 7a, 7b und 16 näher erläutert.

Zunächst ist darauf hinzuweisen, dass die Werkzeugaufnahme 20 innerhalb eines an ein universelles Handstück ankoppelbaren Handstückschafts drehbar gelagert sein muss. Zu diesem Zweck ist vorzugsweise ein Radiallager 50 beispielsweise ein Kugel-, Walz- oder Nadellager mit Innen- und Außenring vorgesehen, das im längsgeschlitzten Drehmoment-Übertragungsbereich 24a auf die Mitnehmerwelle 22 aufgezogen ist und somit gleichzeitig einem Aufspreizen der schnabelförmigen Axialvorsprünge 28 bei einer Drehmomentübertragung auf die Angriffsebenen 8 des Werkzeugschafts 4 entgegenwirkt. Zudem verbessert ein solches Kugellager 50 im längsgeschlitzten Drehmoment-Übertragungsbereich 24a das Einspann-/Ausspannverhältnis am Werkzeugschaft 4, wie dies insbesondere in den Fig. 7a und 7b gezeigt wird.

In der Fig. 7a ist die Einbausituation eines Werkzeugs 1 in einer Werkzeugaufnahme 20 mit Radiallager 50 im Drehmoment-Übertragungsbereich 24a der Mitnehmerwelle 22 gezeigt. Wie hieraus zu entnehmen ist, wird der distal aus dem Handstückschaft 70 herausragende Werkzeugschaft 4 an wenigstens einem distalen Lager (vorzugsweise zwei distale Lager) 72 und an wenigstens einem proximalen Lager 50, 74 abgestützt, um so Werkzeugaufstandskräfte und Schnittkräfte aufzunehmen, die als Biegekräfte auf den Werkzeugschaft 4 wirken. Wird demnach das wenigstens eine proximale Radiallager 50 in den Drehmoment-Übertragungsbereich 24a der Werkzeugaufnahme 22 gelegt, ergibt sich eine Einspannlänge zwischen distalem und proximalem Lager 72, 50 die deutlich größer ist als die Ausspannlänge zwischen distalem Lager 72 und Werkzeugeingriffssegment 2.

In der Fig. 7b ist hingegen ein Bezugsbeispiel gezeigt, dann, wenn als proximal letztes Radiallager das Lager 74 distal zum Drehmoment-Übertragungsbereich 24a angenommen wird. In diesem Fall verkürzt sich die Einspannlänge gegenüber der Ausspannlänge. Es ist offensichtlich, dass im letzteren Fall gemäß der Fig. 7b die Belastung auf die Radiallager 72, 74 vergrößert sind und daher schneller verschleißen. Auch ist die maximal zulässige Belastung kleiner.

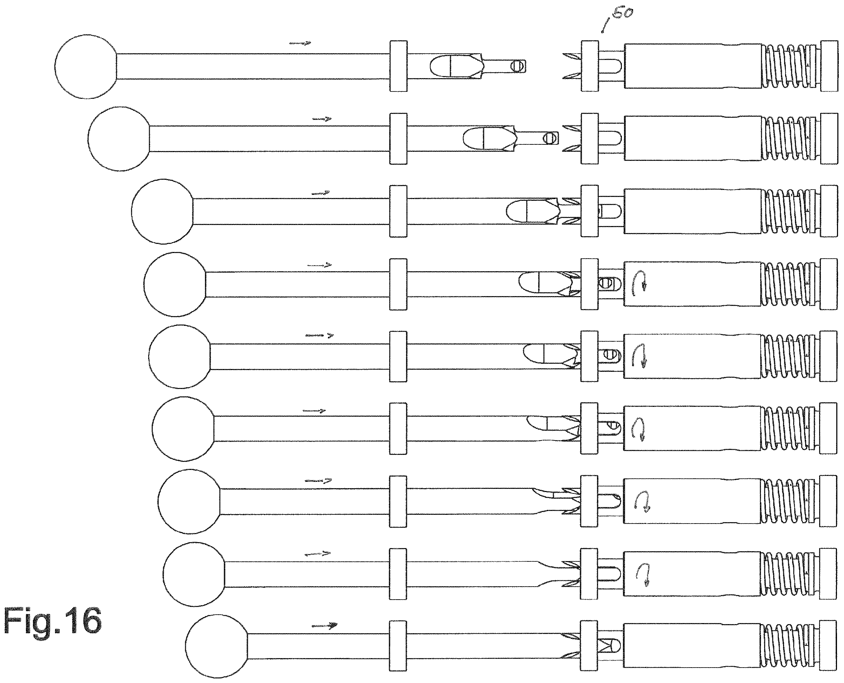

Der Einsetzvorgang des erfindungsgemäßen Werkzeugs 1 in die Werkzeugaufnahme 20 ist im Einzelnen in der Fig. 16 dargestellt.

Zunächst wird der Werkzeugschaft 4 an den Drehmoment-Übertragungsbereich 24a der Werkzeugaufnahme 20 ggf. in einer inkorrekten Relativ-Drehposition herangeführt, wobei in diesem Fall die werkzeugseitigen Abgleitflächen 10 mit den beiden schnabelförmigen Axialvorsprüngen 28 der Werkzeugaufnahme 20 zuerst in Kontakt kommen. Infolge deren Ausrichtung wird die Mitnehmerwelle 22 automatisch gedreht, solange, bis die beiden Angriffsebenen 8 zu den radial äußeren Axialvorsprüngen 28 hinweisen. Jetzt kann der Werkzeugschaft 4 weiter in die Werkzeugaufnahme 20 eingeführt werden, wobei die werkzeugseitigen Angriffsflächen/-ebenen 8 gleitend zwischen den schnabelförmigen Axialvorsprüngen 8 geführt werden. Das in der Fig. 16 ebenfalls gezeigte Radiallager 50 verhindert dabei ein radiales Aufspreizen der schnabel/gabelförmigen Axialvorsprünge/Laschen 28.

Für ein Einsetzen des Werkzeugschafts 4 befindet sich die Schließhülse 46 zunächst in ihrer zurückgezogenen Freigabeposition, in der die Verriegelungskugeln 36 radial nach Außen gedrückt werden können. Letzteres bewerkstelligt das Nachfahrelement 30 (Bolzen), das mit seinem distalen Abschnitt 30a radial zwischen die Verriegelungskugeln 36 durch die Nachfahrfeder 32 gedrückt wird und diese daher radial nach außen hält. Die radial nach außen gedrückten Kugeln 36 halt dann auch die Schließhülse 46 axial in ihrer Freigabeposition.

Beim Eindringen des Werkzeugschafts 4 in die Werkzeugaufnahme 20 stößt jedoch der werkzeugschaftseitige Verriegelungsabschnitt 6b stirnseitig an das Nachfahrelement 30 an und verdrängt dieses in Axialrichtung gegen die Vorspannkraft der Nachfahrfeder 32, solange, bis sich die Taschen/Umfangsnut 12/12a im Verriegelungsabschnitt 6b des Werkzeugschafts 4 im Bereich der Verriegelungskugeln 36 befinden. In diesem Moment werden die Kugeln 36 durch die Schließhülse 46 infolge der axial wirkenden Federvorspannung und einer entsprechenden konischen Formgebung an der Innenumfangsseite der Schließhülse 46 (nicht weiter dargestellt) nach innen gedrückt und kommen somit in der Umfangsnut 12a bzw. den Taschen 12 des Werkzeugschafts 4 zu liegen. Gleichzeitig bewegt sich die Schließhülse 46 durch die Federvorspannung weiter in Richtung nach distal in ihre Verriegelungsposition. Damit ist das Werkzeug 1 axial gesichert und es kann ein Drehmoment von der Torsionswelle 60 über die Mitnahmeelemente 40 und die Mitnehmerwelle 22 auf die Angriffsebenen 8 des Werkzeugschafts 4 übertragen werden.

Um das Werkzeug 1 zu entnehmen, wird die Schließhülse 46 (manuell) gegen die Schließfeder 44 in die Freigabeposition nach proximal zurück gezogen, um die Verriegelungskugeln 36 radial freizugeben. Wird dann der Werkzeugschaft 4 aus der Aufnahme 20 herausgezogen folgt das Nachfahrelement 30 selbsttätig dem Werkzeugschaft 4 infolge der Nachfahrfeder 32 und gelangt so radial zwischen die Verriegelungskugeln 36, um diese radial nach Außen gedrückt zu halten. Die Werkzeugaufnahme 20 verharrt somit in dieser Freigabeposition, um automatisch einen neu eingeführten Werkzeugschaft 4 axial zu verriegeln. Die vorliegende Werkzeugaufnahme 20 kann daher auch als halbautomatische Werkzeugaufnahme (automatisches Verriegeln und manuelles Freigeben) bezeichnet werden.

## Kupplung zwischen Werkzeugaufnahme bzw. Torsionswelle und handstückseitigem Getriebezug

Wie bereits eingangs erläutert wurde, besteht ein Aspekt der vorliegenden Offenbarung darin, für unterschiedliche Handstückschäfte immer das gleiche Werkzeug verwenden zu können. Die Handstückschäfte sind dabei so konstruiert, dass sie an ein einziges, universelles Handstück ankuppelbar sind, in welchem der Werkzeugantrieb und/oder der Drehmoment-Übertragungszug/Getriebe untergebracht ist. Dies bedeutet, dass innerhalb des jeweiligen Handstückschafts eine Torsionswelle vormontiert sein muss, an deren distalem Ende die Werkzeugaufnahme vorzugsweise gemäß der vorstehenden Beschreibung und an deren proximalem Ende eine Kupplung vorgesehen sein muss, die zusammen mit dem festen Ankuppeln des Handstückschafts an das Handstück (vorzugsweise an dessen Gehäuse) gleichzeitig mit dem Drehmoment-Übertragungszug in Wirkeingriff kommt, um eine Drehmomentübertragung auf die Torsionswelle zu ermöglichen.

Eine solche Kupplung muss in erster Linie Drehmomente übertragen, aber auch eine axiale Verschiebung der Antriebswelle ermöglichen, damit beispielsweise die Werkzeugaufnahme entriegelt/demontiert werden kann. Außerdem sollte die Kupplung ausreichende Führungseigenschaften besitzen, um zumindest in diesem Bereich weitere Radiallager (Kugellager) zur Abstützung der Kupplung einsparen zu können.

Bisher wurde die betreffende Kupplung zwischen Torsionswelle (innerhalb des auswechselbaren Handstückschafts) und Drehmomentzug (innerhalb des Handstücks) durch einen sogenannten Zweiflach vergleichbar zum vorstehend beschriebenen erfindungsgemäßen Drehmoment-Übertragungsabschnitt zwischen Werkzeugschaft und Werkzeugaufnahme gebildet. Eine solche Kupplung (ohne umgebendes Radiallager) hat jedoch das grundsätzlich Problem einer zu geringen Torsionssteifigkeit im vorgegebenen (engen) Bauraum, was schnell zu einem Versagen der zusammenwirkenden Kupplungsbauteile führen kann. Außerdem sind mit dieser Konstruktion nur unzureichende Führungseigenschaften erreichbar.

Eine Alternative zu dem genannten Zweiflach bildet die allgemein bekannte Kreuzlösung. In diesem Fall ist der männliche Kupplungsteil auf Seiten der Torsionswelle mit achszentral sich kreuzenden Stegen versehen, die in ein entsprechend geformtes weibliches Kupplungsteil einführbar sind, wodurch die für die Drehmomentübertragung nutzbare Fläche an den Seitenflanken jedes Stegs insgesamt vergrößert wird. Jedoch erweist sich diese Lösung ebenfalls als problematisch, in sofern, als dass hierdurch keine optimale Verteilung der Torsionssteifigkeit zwischen dem männlichen und weiblichen Kupplungsteil vorhanden ist, sodass auch hier das maximal übertragbare Drehmoment begrenzt ist.

Um dieses Problem zu lösen, ist demnach eine Querschnittsgeometrie für die Kupplungsteile (männlich und weiblich) erforderlich, welche den gegebenen Bauraum im Fall eines chirurgischen Handstücks der einschlägigen Gattung optimal bezüglich Torsionsträgheitsmoment ausnutzt, gleichzeitig eine axiale Verschiebung der beiden Kupplungsbauteile relativ zueinander ermöglicht und durch die besondere Form nicht in eine Selbsthemmung gerät.

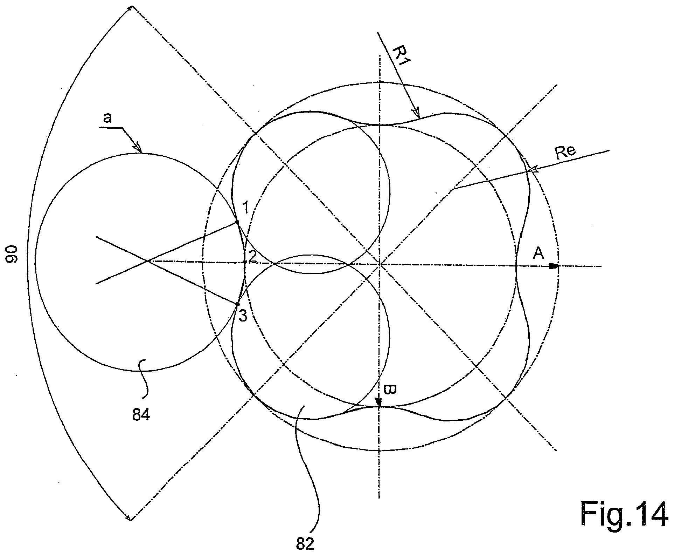

Bei der Entwicklung der Kupplung hat sich dabei ergeben, dass je kreisähnlicher die Mitnehmerkontur wird, desto weniger Formschluss ist für eine Drehmomentübertragung vorhanden. Aber auch je weniger Ecken die Mitnehmerkontur hat, desto formschlüssiger verbleibt die Kupplungspaarung (bei abnehmendem Widerstandsmoment). Insgesamt erweist sich daher bei einem chirurgischen Instrument dieser Gattung eine Kleeblattkupplung 80 mit vier Blättern als besonders vorteilhafte Kupplungsquerschnittsform. In der Fig. 14 ist ein optimierter Querschnitt einer vierblättrigen Kleeblattform gemäß einem bevorzugten Ausführungsbeispiel der Offenbarung dargestellt.

Demzufolge wird die Querschnittsform der Kupplung 80 durch vier gleiche Kreise 82 mit kleinerem Radius Re aufgebaut, welche die vier Ecken der Kupplungsform definieren und die jeweils um 90° winkelversetzt zueinander angeordnet sind. Der Abstand der jeweils benachbarten innerhalb der Kupplungsform liegenden Kreismittelpunkte ist geringfügig kleiner als der einheitliche Kreisdurchmesser Re, sodass sich die jeweils benachbarten Kreise 82 schneiden.

Auf einer Mittelachse zwischen zwei benachbarten, sich schneidenden Kreisen 82 ist außerhalb der Kupplungsform jeweils ein weiterer Kreismittelpunkt gesetzt, wobei um diesen ein Kreis 84 mit größerem Radius Ri gezogen ist. Die jeweilige Position dieses äußeren, weiteren Kreismittelpunkts sowie der größere Radius Ri sind so gewählt, dass die Kontur des äußeren Kreises 84 kontinuierlich in die Kontur der beiden eckseitigen inneren Kreise 82 übergeht und somit unter Ausbildung einer Kavität beide jeweils benachbarten Eckkreise 82 miteinander verbindet. D.h. der äußere Kreis 84 verläuft in den Kontaktpunkten zu den beiden inneren Eckkreisen 82 jeweils tangential, wodurch eine stetige (ecken-/kantenlose) Querschnittskontur mit vier ausgeprägt konvexen Eckkreisen 82 und vier sanft konkaven Seitenkreisen 84 entsteht.

Geometrisch lässt sich die Querschnittskontur gemäß dem bevorzugten Ausführungsbeispiel wie Folgt definieren:

Gemäß der Fig. 14 bedeutet der Wert A den Durchmesser eines Unfangskreises mit querschnittsform-zentrischen Kreismittelpunkt und radial äußeren Berührpunkten an allen Eckkreisen. Der Wert B bedeutet das lichte Maß zwischen zwei sich gegenüberliegenden Seitenkreisen, d.h. der Diagonalabstand der querschnittsforminnersten Punkte zweier gegenüberliegender Seitenkreise.

Demnach stehen diese Werte A, B in einem Verhältnis zueinander gemäß der Formel (1):

$$(1) \quad B = kB * A \qquad mit \qquad 0,6 < kB < 0,9$$

Der Radius Re jedes Eckkreises steht in einem Verhältnis zum Wert A gemäß der Formel (2):

$$(2) \quad Re = kRe * A \qquad mit \qquad 0,6 < kRe < 0,9$$

Der Radius Ri jedes Seitenkreises steht in einem Verhältnis zum Wert A gemäß der Formel (3):

$$(3) \quad Ri = kRi * A \qquad mit \qquad 0,8 < kRi < 1,5$$

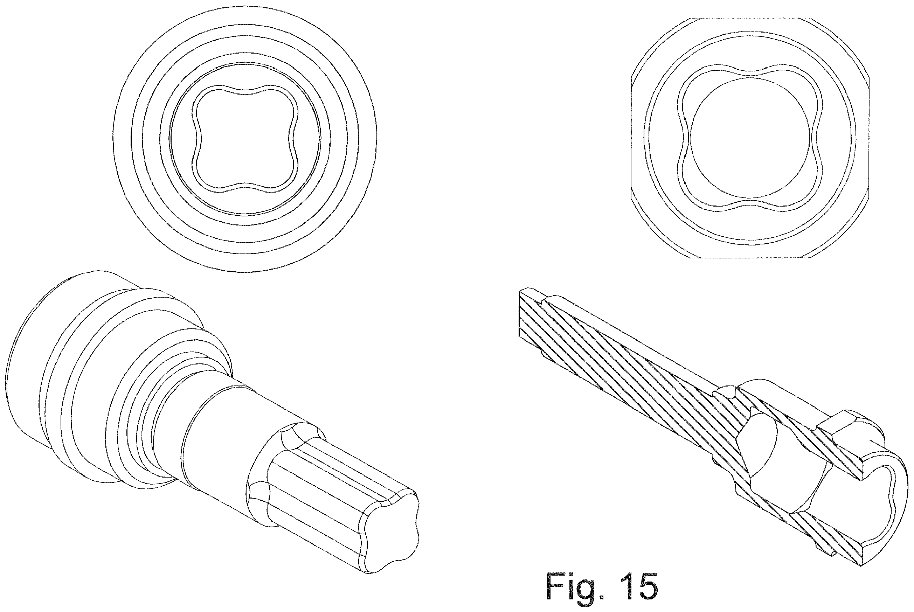

Wie die Fig. 15 zeigt, weisen sowohl der männliche wie auch der weibliche Kupplungsteil eine entsprechende Querschnittsform auf, mit einem bestimmten Übermaß des weiblichen Kupplungsteils.

**Patentansprüche**

1. Spanabhebendes Werkzeug eines chirurgischen, Drehmoment übertragenden Instruments mit einem distalen Eingriffssegment (2), an das sich ein Werkzeugschaft (4) anschließt, dessen proximaler Endabschnitt (6) für ein Drehmoment übertragendes Einsetzen in eine Werkzeugaufnahme (20) des Instruments vorbereitet ist, wofür der proximale Endabschnitt (6) zumindest in einen Funktionsabschnitt "Drehmoment-Übertragung" (6a) und einen axial hiervon beabstandeten Funktionsabschnitt "Axialverriegelung" (6b) unterteilt ist, wobei sich der Funktionsabschnitt "Axialverriegelung" (6b) bezüglich des Eingriffssegments (2) proximal zum Funktionsabschnitt "Drehmoment-Übertragung" (6a) anordnet, mit einem weiteren Funktionsabschnitt "Eindrehhilfe" vorzugsweise als Bestandteil des Funktionsabschnitts "Drehmoment-Übertragung" (6a), **dadurch gekennzeichnet, dass** der Funktionsabschnitt "Eindrehhilfe" axial zwischen den Funktionsabschnitten "Drehmoment-Übertragung" (6a) und "Axialverriegelung" (6b) vorzugsweise im Bereich eines Radialabsatzes (6c) zwischen den Funktionsabschnitten, Drehmoment-Übertragung" (6a) und "Axialverriegelung" (6b) vorgesehen ist; und dass der Funktionsabschnitt "Drehmoment-Übertragung" (6a) mit einem Zweiflach ausgebildet ist, dessen zwei diametral voneinander abgewandte Angriffsebenen (8) vorzugsweise keilförmig in Richtung Funktionsabschnitt "Axialverriegelung" (6b) aufeinander zulaufen, und der Funktionsabschnitt "Eindrehhilfe" eine Anzahl, vorzugsweise vier, keilförmig ausgerichtete Abgleitebenen (10) hat, von denen jeweils zwei Abgleitflächen (10) zu beiden Längsseiten jeweils einer Angriffsebene (8) in einem Winkel zu dieser ausgebildet sind und dabei die Längskanten der jeweiligen Angriffsebene (8) zumindest axialabschnittsweise brechen.

2. Spanabhebendes Werkzeug nach Anspruch 1, **dadurch gekennzeichnet, dass** der Werkzeugschaftdurchmesser d im Funktionsabschnitt "Axialverriegelung" (6b) kleiner ist als der Werkzeugschaftdurchmesser D im Funktionsabschnitt "Drehmoment-Übertragung" (6a), wobei sich zwischen beiden Funktionsabschnitten vorzugsweise ein Radialabsatz (6c) ausbildet.

3. Spanabhebendes Werkzeug nach Anspruch 2, **dadurch gekennzeichnet, dass** für die beiden Werzeugschaftdurchmesser gilt: d <= 0,6 D

4. Spanabhebendes Werkzeug nach Anspruch 1, **dadurch gekennzeichnet, dass** die Angriffsebenen (8) durch eine entsprechend plane Verringerung des Schaftdurchmessers D im Funktionsabschnitt "Drehmoment-Übertragung" (6a) hergestellt sind.

5. Spanabhebendes Werkzeug nach einem der vorstehenden Ansprüche, **dadurch gekennzeichnet, dass** der Funktionsabschnitt "Axialverriegelung" (6b) vorzugsweise an seinem proximalen Ende eine Anzahl, weiter vorzugsweise zwei radialer Einbuchtungen (12) oder eine Umfangsnut (12a) hat, wodurch axial wirkende Hinterschneidungen am Werkzeugschaftumfang gebildet werden.

6. Chirurgisches, Drehmoment übertragendes Instrument mit einem drehbar gelagerten chirurgischen Werkzeug (1) und einer Werkzeugaufnahme (20) für ein wahlweises Aufnehmen des Werkzeugs (1) zur axialen Sicherung des Werkzeugs (1) in der Werkzeugaufnahme (20) sowie zur Übertragung eines Drehmoments auf das Werkzeug (1), **dadurch gekennzeichnet, dass** das Werkzeug (1) die technischen Merkmale gemäß einem der Ansprüche 1 bis 5 aufweist.

7. Chirurgisches, Drehmoment übertragendes Instrument nach Anspruch 6, **gekennzeichnet durch** ein universelles Handstück mit integriertem Drehmoment-Übertragungszug und/oder Antrieb und einem auswählbaren Handstückschaft (70) mit integrierter Werkzeugaufnahme (20), der an das universelle Handstück anflanschbar ist unter Kopplung der Werkzeugaufnahme (20) an den Drehmoment-Übertragungszug oder Antrieb.

## Claims

1. A chip-removing tool of a surgical torque-transferring instrument comprising a distal engagement segment (2) adjoined by a tool shaft (4) whose proximal end portion (6) is prepared for a torque-transferring insertion into a tool mounting (20) of the instrument, for which purpose the proximal end portion (6) is subdivided at least in a functional section "torque transmission" (6a) and a functional section "axial locking" (6b) axially spaced therefrom, the functional section "axial locking" (6b) being arranged with respect to the engagement segment (2) so as to be proximal relative to the functional section "torque transmission" (6a), with a further functional section "screw-in aid" preferably as a part of the functional section "torque transmission" (6a), **characterized in that**
the functional section "screw-in aid" is axially provided between the functional sections "torque transmission" (6a) and "axial locking" (6b) preferably in the region of a radial shoulder (6c) between the functional sections "torque transmission" (6a) and "axial locking" (6b),and that the functional section "torque transmission" (6a) is designed so as to comprise a dihedron whose two contact planes (8) diametrically facing away from each other extend toward each other preferably in a wedge-shaped manner toward the functional section "axial locking" (6b), and the functional section "screw-in aid" comprises a number of glide planes (10), preferably four of them, which are oriented in a wedge-shaped manner, two of which being formed at both longitudinal sides of a respective one of said contact planes (8) at an angle relative thereto and breaking the longitudinal edges of the respective contact planes (8) at least in axial portions.

2. The chip-removing tool according to claim 1, **characterized in that** the tool shaft diameter d in the functional section "axial locking" (6b) is smaller than the tool shaft diameter D in the functional section "torque transmission" (6a), a radial shoulder (6c) being preferably formed between said two functional sections.

3. The chip-removing tool according to claim 2, **characterized in that** d <= 0,6 D applies to both tool shaft diameters.

4. The chip-removing tool according to claim 1, **characterized in that** the contact planes (8) are manufactured by a correspondingly planar reduction of the shaft diameter D in the functional section "torque transmission" (6a).

5. The chip-removing tool according to any of the preceding claims, **characterized in that** the functional section "axial locking" (6b), preferably at its proximal end, comprises a number of radial indentations (12), more preferably two of them, or a circumferential groove (12a), whereby axially acting undercuts are formed on the tool shaft circumference.

6. A surgical torque-transferring instrument comprising a rotatably supported surgical tool (1) and a tool mounting (20) for selectively receiving the tool (1) for axially securing the tool (1) in the tool mounting (20) as well as for transferring a torque to the tool (1), **characterized in that** the tool (1) comprises the technical features according to any one of claims 1 to 5.

7. A surgical torque-transferring instrument according to claim 6, **characterized by** a universal handpiece comprising an integrated torque transmission train and/or a drive and a selectable handpiece shaft (70) comprising an integrated tool mounting (20), said handpiece shaft being able to be flanged to the universal handpiece while coupling the tool mounting (20) to the torque transmission train or the drive.

**Revendications**

1. Outil à enlèvement de copeaux d'un instrument chirurgical transmettant un couple de rotation avec un segment de prise distale (2), auquel se raccorde un manche d'outil (4), dont la section d'extrémité proximale (6) est préparée en vue d'une insertion avec transmission de couple de rotation dans un logement d'outil (20) de l'instrument, la section d'extrémité proximale (6) étant divisée à cet effet au moins en une section fonctionnelle « transmission de couple de rotation » (6a) et en une section fonctionnelle « verrouillage axial » (6b), espacée de manière axiale de cette dernière, dans lequel la section fonctionnelle « verrouillage axial » (6b) s'agence par rapport au segment de prise (2) de manière proximale par rapport à la section fonctionnelle « transmission de couple de rotation » (6a), avec une autre section fonctionnelle « assistance à la rotation », de préférence en tant qu'élément constitutif de la section fonctionnelle « transmission de couple de rotation » (6a),
caractérisé en ce que la section fonctionnelle « assistance à la rotation » est prévue de manière axiale entre les sections fonctionnelles « transmission de couple de rotation » (6a) et « verrouillage axial » (6b) de préférence dans la zone d'un décrochement radial (6c) entre les sections fonctionnelles « transmission de couple de rotation » (6a) et « verrouillage axial » (6b) ; et que la section fonctionnelle « transmission de couple de rotation » (6a) est réalisée avec un dièdre, dont les deux plans d'attaque (8) diamétralement opposés l'un de l'autre convergent l'un vers l'autre de préférence de manière cunéiforme en direction de la section fonctionnelle « verrouillage axial » (6b), et la section fonctionnelle « assistance à la rotation » a un nombre donné, de préférence quatre, plans de glissement (10) orientés de manière cunéiforme, dont respectivement deux surfaces de glissement (10) sont réalisées par rapport aux deux côtés longitudinaux respectivement d'un plan d'attaque (8) selon un angle par rapport à ce dernier et cassent ce faisant au moins par endroits axiaux les arêtes longitudinales du plan d'attaque (8) respectif.

2. Outil à enlèvement de copeaux selon la revendication 1, caractérisé en ce que le diamètre de manche d'outil d dans la section fonctionnelle « verrouillage axial » (6b) est inférieur au diamètre de manche d'outil D dans la section fonctionnelle « transmission de couple de rotation » (6a), dans lequel de préférence un décrochement radial (6c) se forme entre les deux sections fonctionnelles.

3. Outil d'enlèvement de copeaux selon la revendication 2, caractérisé en ce que s'applique pour les deux diamètres de manche d'outil : d <= 0,6 D.

4. Outil d'enlèvement de copeaux selon la revendication 1, caractérisé en ce que les plans d'attaque (8) sont fabriqués par une réduction plane de manière conforme du diamètre de manche D dans la section fonctionnelle « transmission de couple de rotation » (6a).

5. Outil d'enlèvement de copeaux selon l'une quelconque des revendications précédentes, caractérisé en ce que la section fonctionnelle « verrouillage axial » (6b) a, de préférence au niveau de son extrémité proximale, un nombre donné, de préférence encore deux indentations (12) radiales ou une rainure périphérique (12a), ce qui permet de former des contre-dépouilles agissant de manière axiale au niveau de la périphérie de manche d'outil.

6. Instrument chirurgical à transmission de couple de rotation avec un outil (1) chirurgical monté de manière à pouvoir tourner et un logement d'outil (20) pour un logement au choix de l'outil (1), servant à bloquer de manière axiale l'outil (1) dans le logement d'outil (20) et servant également à transmettre un couple de rotation sur l'outil (1), caractérisé en ce que l'outil (1) présente les caractéristiques techniques selon l'une quelconque des revendications 1 à 5.

7. Instrument chirurgical à transmission de couple de rotation selon la revendication 6, caractérisé par une pièce à main universelle avec un câble de transmission de couple de rotation intégré et/ou un entraînement et un manche de pièce à main (70) pouvant être choisi avec un logement d'outil (20) intégré, qui peut être installé par bride au niveau de la pièce à main universelle avec un couplage du logement d'outil (20) au câble de transmission de couple de rotation ou à l'entraînement.

FIG. 1

Fig.2

Fig.4a

Fig.3

Fig.4b

Fig.5

Fig.6

Fig.7a

Fig.7b

Fig.8

EP 2 892 442 B1

Fig.9

Fig.10

Fig.11

Fig.12

Fig.13

Fig.14

Fig. 15

Fig.16

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1605837 A1
- US 2011098688 A1
- US 20030023256 A1
- DE 10311455 B3